(19) Europäisches Patentamt
European Patent Office
Office européen des brevets

(11) Veröffentlichungsnummer : **0 359 187 B1**

(12) **EUROPÄISCHE PATENTSCHRIFT**

(45) Veröffentlichungstag der Patentschrift : **20.05.92 Patentblatt 92/21**

(51) Int. Cl.[5] : **C07D 493/10,** C07D 309/10, A61K 31/35

(21) Anmeldenummer : **89116830.4**

(22) Anmeldetag : **12.09.89**

# (54) Basische Spaltungsprodukte von Elaiophylin und Elaiophylinderivaten und Verwendung derselben.

(30) Priorität : **16.09.88 DE 3831465**

(43) Veröffentlichungstag der Anmeldung : **21.03.90 Patentblatt 90/12**

(45) Bekanntmachung des Hinweises auf die Patenterteilung : **20.05.92 Patentblatt 92/21**

(84) Benannte Vertragsstaaten : **BE CH DE FR GB IT LI NL**

(56) Entgegenhaltungen :
**CHEMICAL ABSTRACTS, Band 104, Nr. 13, 31. März 1986, Columbus, Ohio, US; JACKSON, R.F.W.; SUTTER, M.A.; SEEBACH, D.:"Preparation of (2E, 4E, 6S, 7S, 10E, 12E, 14S, 15S, 1'S)-7,15-bis(1'-hydroxy-methylethyl--6,14-dimethyl-8,16-dioxa-2,4,10,12-cyclohexadecatetraene-1,19-dione. A building blook for the synthesis ofelaiophylin" Seite 694, Spalte 2, Zusammen-fassung-Nr. 109 317a**
**CHEMICAL ABSTRACTS, Band 107, Nr. 9, 31. August 1987, Columbus, Ohio, US; TOSHIMA, K.; TATSUTA, K.; KINOSHITA, M.: "Totalsynthe- sis of elaiphylin (azalomycin B)" Seite 739, Spalte 2, Zusammen-fassung-Nr. 78 160z**

(56) Entgegenhaltungen :
**CHEMICAL ABSTRACTS, Band 110, Nr. 9, 27. Februar 1989, Columbus, Ohio, US; TOSHIMA, K.; TATSUTA, K.; KINOSHITA, M.: "Totalsynthe- sis of elaiophylin (azalomycin B)" Seite 696, Spalte 1, Zusammenfassung-Nr. 75 912x**
**CHEMICAL ABSTRACTS, Band 103, Nr. 15, 14. Oktober 1985, Columbus, Ohio, US; SEEBACH, D.; CHOW, H.F.; JACKSON, R. et al.:"Total synthesis of (+)-11,11'-di-O- methylelaiophylidene, an aglucon of elaiophylin." Seite 702, Spalte 2, Zusammenfassung-Nr. 123227g**

(73) Patentinhaber : **HOECHST AKTIENGESELLSCHAFT
Postfach 80 03 20
W-6230 Frankfurt am Main 80 (DE)**

(72) Erfinder : **Kretzschmar, Gerhard, Dr.
Ulmenweg 10
W-6236 Eschborn 2 (DE)**
Erfinder : **Hammann, Peter, Dr.
Mörikestrasse 6
W-6233 Kelkheim(Taunus) (DE)**
Erfinder : **Düwel, Dieter, Dr.
Frankfurter Strasse 39
W-6238 Hofheim am Taunus (DE)**
Erfinder : **Wöhner, Gerhard, Dr.
Mühlenkampstrasse 77
W-3050 Wunstorf (DE)**
Erfinder : **Marquardt, Rüdiger, Dr.
Günthersburgallee 69
W-6000 Frankfurt am Main 60 (DE)**
Erfinder : **Kühlein, Klaus, Prof. Dr.
Fasanenstrasse 41
W-6233 Kelkheim(Taunus) (DE)**

**EP 0 359 187 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Makrocyclen mit 16-gliedriger Lactonstruktur sind als anthelminthische Wirkstoffe von besonderem wirtschaftlichem Interesse. Bekannte Vertreter aus dieser Wirkstoffklasse sind die Avermectine und Milbemycine (Übersicht in: Spec. Publ. - R. Soc. Chem. 53, Recent Advances Chem. Insect Control, 1985) sowie die Bafilomycine (G. Werner et al., J. Antibiot. 37, 110 (1984)), das Tubaymycin (DE 34 02 075 A1) und Leucanicidin (JP 60,190,785).

Das Makrolid-Antibiotikum Elaiophylin wurde erstmals von Arcamone et al. 1959 (Giorn. Microbiol. 7, 207, 1959) aus Kulturen von Streptomyces melanosporus isoliert und später von Arai unter dem Namen Azalomycin B beschrieben (J. Antibiot., Ser. A, 13, 46, 51 (1960)).

Ein stark verbessertes Verfahren zur Herstellung von Elaiophylin und dessen Verwendung als Anthelmintikum wird in der deutschen Patentanmeldung P 37.21.722.4 (EP-A-297 523) vorgeschlagen.

Die Struktur von Elaiophylin wurde durch spektroskopische Untersuchungen und Abbaureaktionen von Kaiser et al. (Helv. Chim. Acta, 64 (1981), 407) sowie durch Röntgenbeugung (S.V. Ley et al., Tetrahedron Lett., 1207 (1982)) eindeutig zugeordnet.

Die interessanten biologischen Eigenschaften und die ungewöhnliche $C_2$-Symmetrie dieser 16-gliedrigen Makrodiolidverbindung stimulierten auch synthetische Arbeiten zum enantioselektiven Aufbau des Makrocyklus als Modellstruktur (D. Seebach et al., Liebigs Ann. Chem. 1983, 939 und T. Wakamatsu et al., Heterocycles 25, 43, 1987). Diese Arbeiten führten schließlich zur Totalsynthese des Aglykons 11,11'-Di-O-Methylelaiophyliden (D. Seebach et al., J. Am. Chem. Soc., 107, 5292, 1985) und der naturidentischen Verbindung durch M. Kinoshita et al. (Tetrahedron Lett. 1986, 4741).

Während Elaiophylin durch Behandlung mit Säuren in definierter Weise über das isolierbare Aglykon abgebaut werden kann, führt die Umsetzung mit milden Basen zur vollständigen, unkontrollierten Zersetzung des Moleküls (D. Seebach et al., Liebigs. Ann. Chem. 1281, 1986).

Führt man jedoch zunächst eine in-situ-Derivatisierung des Elaiophylins oder von Elaiophylinderivaten zu den 11,11'-Di-O-Alkylderivaten durch und setzt anschließend mit Alkoholaten um, so erhält man in hohen Ausbeuten die von der symmetrischen Makrodiolidstruktur abgeleiteten seco-Elaiophylin-Ester. Mit dem gleichen Erfolg können auch die isolierten 11,11'-Di-O-Alkylderivate für die Spaltreaktion eingesetzt werden. Einige 11,11'-O-Alkylderivate sind bereits in der Japanischen Offenlegungsschrift 61-36295 (Yokura et al.) beschrieben. Für deren Herstellung wird ein verbessertes Verfahren mittels Metallsalz-Katalyse in der deutschen Patentanmeldung P 37 36 960.1 (EP-A-315 003) vorgeschlagen.

Es wurde nun überraschend gefunden, daß nicht nur der Makrocyclus Elaiophylin selbst als Anthelminthikum verwendet werden kann, sondern daß auch die mittels basischem Abbau erstmals hergestellten identischen seco-Hälften des Naturstoffs und deren Derivate eine anthelminthische Aktivität besitzen, die der von Elaiophylin selbst vergleichbar ist.

Die Erfindung betrifft nun:

1. Elaiophylinderivate der Formeln (I) und (III)

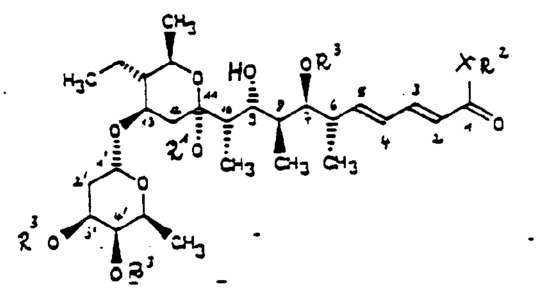

I

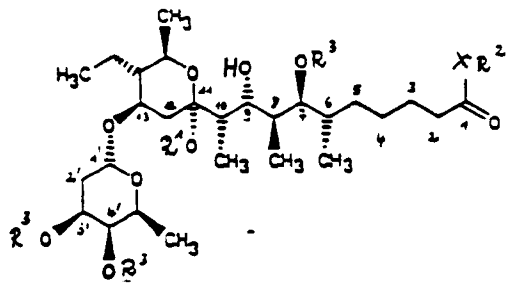

I'

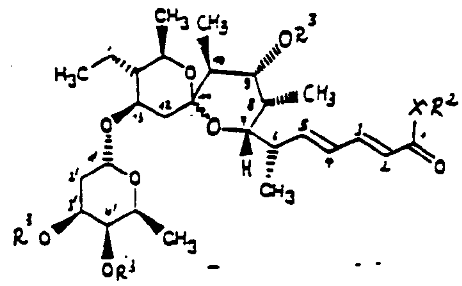

III

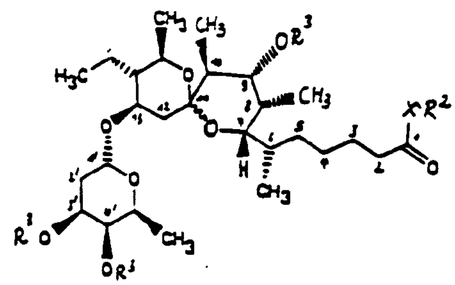

III'

worin die C=C Doppelbindungen in 2,3 und 4,5 Position auc hydriert sein können (Formeln (I') und (III')) und in denen

$R^1$ Wasserstoff oder ein Rest der Formel (VI)

$$-(CH_2)_n-R^4 \qquad (VI)$$

wobei

n = 1 bis 3 und

$R^4$ Wasserstoff, $C_1$-$C_{15}$-Alkyl, $C_2$-$C_{15}$-Alkenyl, $C_2$-$C_{15}$-Alkinyl, $C_3$-$C_9$-Cycloalkyl, Phenyl oder ein Heteroaryl mit 3 bis 9 Ringatomen ist, wobei der Heterocyklus gegebenenfalls Chlor, Brom, Jod, nitro-, hydroxy- oder $C_1$-$C_4$-alkyl- oder $C_1$-$C_4$-alkoxy-substituiert ist,

X 0 oder, wenn $R^1$ nicht Wasserstoff ist und die C=C Doppelbindungen in Formel (I) und (III) in 2,3 und 4,5 Position hydriert sind, auch NH bedeutet,

$R^2$ Wasserstoff oder $C_1$-$C_4$-Alkyl ist oder, falls X NH ist, ein Rest mit der Formel VI ist, wobein und $R^4$ die oben angegebenen Bedeutungen haben und

$R^3$ Wasserstoff oder ein Rest der Formel (II)

$$-\overset{O}{C}-(CH_2)_n-R^4 \qquad (II)$$

bedeutet, worin n und $R^4$ die oben angegebenen Bedeutungen haben.

Weiterhin betrifft die Erfindung ein Verfahren zur Herstellung von Elaiophylinderivaten der Formeln I, III, I'

und III′, das dadurch gekennzeichnet ist, daß man

a) Elaiophylin der Formel (IV), worin $R^1$, $R^2$ und $R^3$ Wasserstoff bedeuten, mit einem Alkohol der Formel $HO-(CH_2)_n-R^4$, worin n und $R^4$ die oben zu Formel IV angegebenen Bedeutungen haben, umsetzt zu einer Verbindung der Formel (IVa) in der $R^1$ $(CH_2)_n-R^4$ bedeutet, wobein und $R^4$ die oben zu Formel VI angegebenen Bedeutungen haben und das so erhaltene Zwischenprodukt (IVa), gegebenenfalls nach seiner Isolierung, entweder zunächst

b) in Gegenwart von Hydrierkatalysatoren zu der Verbindung (IVa′) reduziert, in der die C=C Doppelbindungen in Position 2,3 und 4,5 sowie 2′,3′ und 4′,5′ hydriert sind und $R^1$ gegenüber der Verbindung der Formel IVa unverändert ist, oder

c) das nach Reaktion a) erhältliche Zwischenprodukt IVa oder das nach Reaktion b) erhältliche Zwischenprodukt IVa′ mit Alkalimetallalkoholaten der Formel $R^2O^-M^+$, worin M Lithium, Natrium oder Kalium bedeuten und $R^2$ die oben zu Formel VI angegebene Bedeutung hat, umsetzt zu Verbindungen der Formel Ic bzw. Ic′, worin $R^3$ Wasserstoff, $XR^2$ einen Rest $OR^2$ und $R^1$ und $R^2$ außer Wasserstoff die oben zu Formel VI angegebenen Bedeutungen haben, oder

d) eine Verbindung der Formel Ic in einem nichtwäßrigen Lösungsmittel umsetzt mit einer LEWIS-Säure, zu einer Verbindung der Formel (IIIc), in der die Bedeutung von $XR^2$ und $R^3$ gegenüber der Ausgangsverbindung der Formel (Ic) unverändert bleibt, oder

e) eine Verbindung der Formel (Ic) oder (IIIc) mit Wasserstoff in Gegenwart von Hydrierkatalysatoren zu Verbindungen der Formeln (Ic′) oder (IIIc′) reduziert, worin $R^1$, $XR^2$ und $R^3$ gegenüber der Verbindung der Formeln Ic oder IIIc unverändert bleiben, oder

f) eine Verbindung der Formel Ic mit Hydrierkatalysatoren, die zusätzlich eine LEWIS-Säure Aktivität aufweisen zu einer Verbindung der Formel IIIc′ reduziert, oder

g) eine Verbindung der Formel Ic′ oder IIIc′ umsetzt mit Ammoniak oder einem primären Amin der Formel $H_2N-(CH_2)_n-R^4$, in der n und $R^4$ die oben zu Formel VI angegebenen Bedeutungen haben, zu Verbindungen der Formel Ie′ oder IIIe′ in denen X NH bedeutet, oder

h) eine Verbindung der Formeln Ic, Ic′, IIIc, IIIc′, Ie′ oder IIIe′ umsetzt mit einer Verbindung der FormelV

$$Z-CO-(CH_2)-R^4 \qquad (V)$$

worin n und $R^4$ die zu Formel II angegebenen Bedeutungen haben und in der Z eine nucleofuge Gruppe bedeutet zu einer Verbindung der Formeln (I) oder (III) bzw. (I′) oder (III′), worin $R^1$, $XR^2$ und $R^3$ die oben zu den Formeln I, I′, III und III′ angegebenen Bedeutungen haben, oder

i) eine Verbindung der Formel (I) oder (I′) worin $R^1$ und $R^2$, bis auf Wasserstoff, sowie $R^3$ die oben zu Formel VI angegebenen Bedeutungen haben, in Gegenwart eines wäßrigen Lösungsmittels mit einer LEWIS-Säure umsetzt, wobei man eine Verbindung der Formel (I) oder (I′) erhält, in der $R^1$ Wasserstoff bedeutet und $XR^2$ und $R^3$ gegenüber der Ausgangsverbindung (I) oder (I′) für die Reaktion i) unverändert bleiben.

Außerdem betrifft die Erfindung die Elaiophylinderivate wie oben definiert, zur Verwendung als Arzneimittel, insbesondere als anthelmintisch wirkende Arzneimittel.

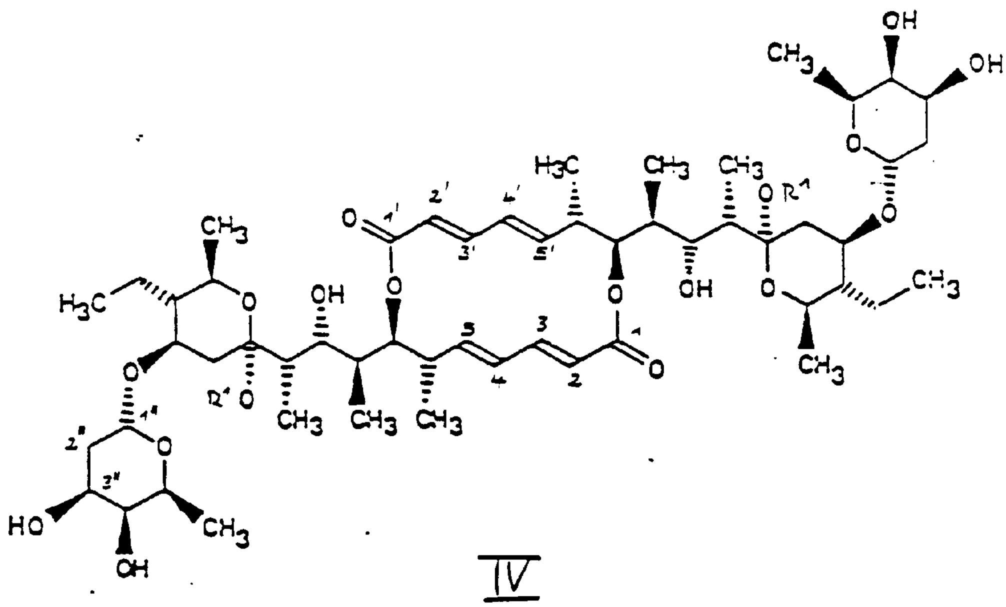

IV

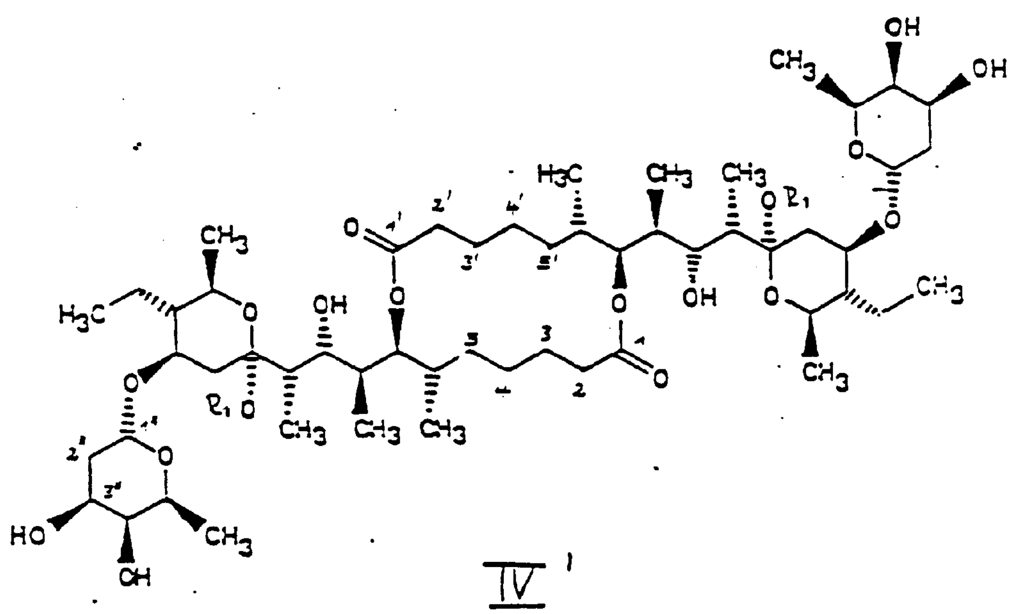

IV'

Reaktionsschema

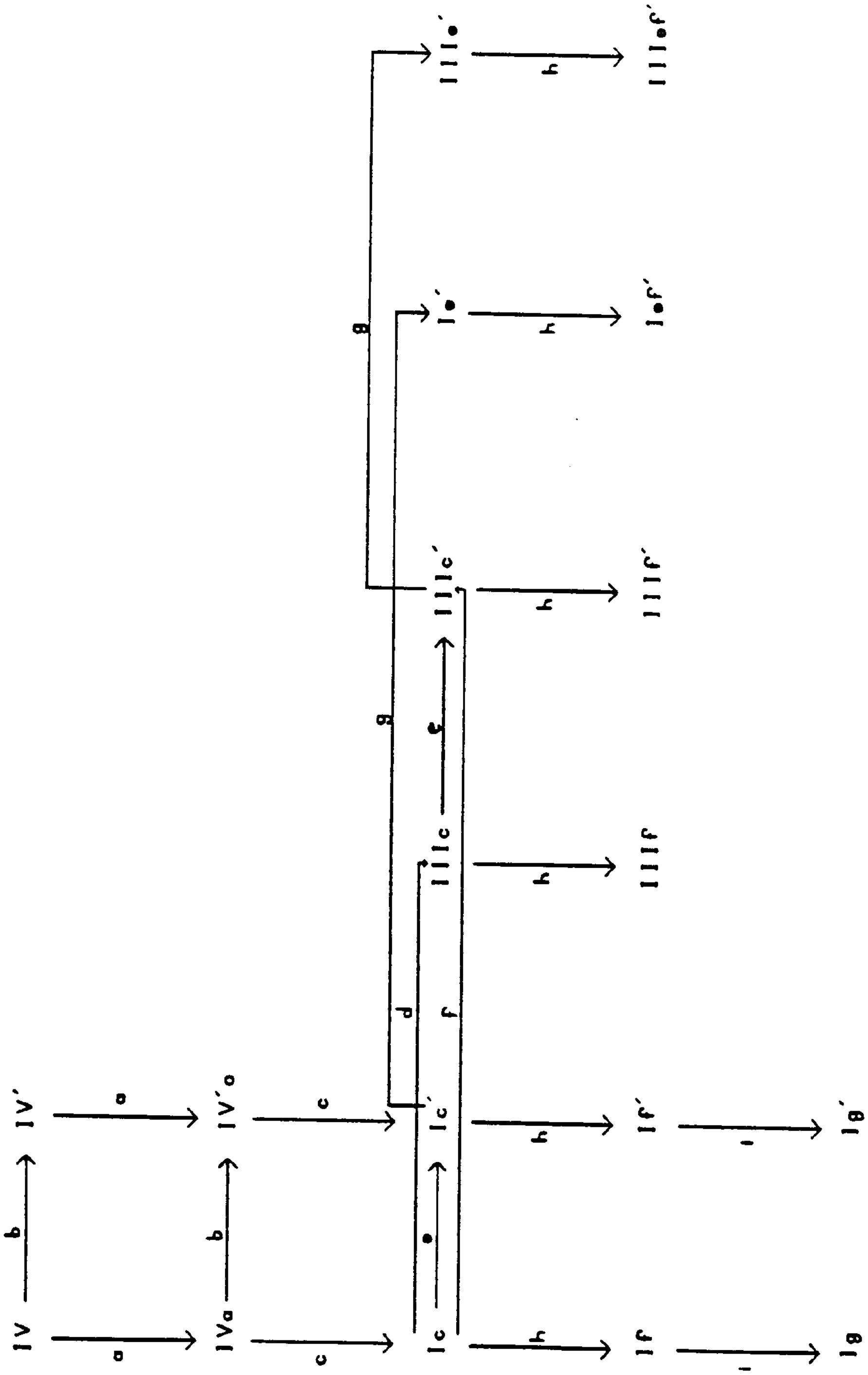

| Verbindung | Generelle Formel | $R^1$ | X | $R^2$ | $R^3$ |
|---|---|---|---|---|---|
| IV | IV | H | - | - | - |
| IV' | IV' | H | - | - | - |
| IVa | IV | R | - | - | - |
| IVa' | IV' | R | - | - | - |
| Ic | I | R | O | R | H |
| Ic' | I' | R | O | R | H |
| IIIc | III | - | O | R | H |
| IIIc' | III' | - | O | R | H |
| Ie' | I' | R | NH | R bzw. H | H |
| IIIe' | III' | - | NH | R bzw. H | H |
| If | I | R | O | R | CO-R |
| If' | I' | R | O | R | CO-R |
| IIIf | III | - | O | R | CO-R |
| IIIf' | III' | - | O | R | CO-R |
| Ief' | I' | R | NH | R bzw. H | CO-R |
| IIIef' | III' | - | NH | R bzw. H | CO-R |
| Ig | I | H | O | R | CO-R |
| Ig' | I' | H | O | R | CO-R |

R bedeutet $(CH_2)_n$-$R^4$ wobei n und $R^4$ die in Anspruch 1 angegebenen Bedeutungen haben.

Bei den Hydrierverfahren b) und e) ist zu beachten, daß mit Wasserstoff reduzierbare funktionelle Gruppen in den Resten $R^1$, $XR^2$ und $R^3$ der jeweils zu hydrierenden ungesättigten Verbindung der allgemeinen Formeln (I), (III) und (IV) gleichzeitig mitreduziert werden können, was gegebenenfalls eine Vertauschung der Reihenfolge der Verfahrensschritte erforderlich machen kann, sofern diese Hydrierung in der Seitenkette nicht erwünscht ist. So können z.B. die Reste $R^1$, falls sie gleichzeitig hydrierbare funktionelle Gruppen besitzen, mittels Verfahren a) auch erst nach Durchführung der Verfahrensstufen b) oder e) eingeführt werden und hydrierbare Reste $R^3$ werden zweckmäßig mittels Verfahren h) erst nach der Verfahrensstufe e) eingeführt. Analoge Überlegungen gelten auch, falls $R^1$ oder $R^3$ den Hydrierkatalysator vergiftende funktionelle Gruppen tragen, wie z.B. in Schwefelverbindungen.

Unter Heteroaryl werden heteroaromatische Kohlenwasserstoffe, insbesondere Thiophen und Furan, aber auch Pyridin, Pyrimidin und Pyrazin verstanden. Die C-1- bis C-10-seitenkette der Verbindungen der Formel I kann - unabhängig von der Art der Substituenten $R^1$ und $R^3$ - hydriert oder nicht hydriert sein. Dies gilt auch für den Rest $XR^2$, sofern X Sauerstoff bedeutet. Falls X = NH bedeutet, liegen die Verbindungen der Formeln I und II in der hydrierten Form vor.

Im folgenden werden die Verfahren a) bis i), die es ermöglichen die unterschiedlich substituierten Verbindungen der Formeln (I) und (III) darzustellen, näher beschrieben.

Mit Hilfe des Verfahrens a) läßt sich der Substituent $R^1$ in Elaiophylin (IV, $R^1$ = H) oder in 2,2′,3,3′,4,4′,5,5′-Octahydroelaiophylin (IV, $R^1$ = H, C=C-Doppelbindungen hydriert, beispielsweise nach Verfahren b)) einführen. Dies ist zur Durchführung der Spaltreaktion nach Verfahren c) zweckmäßig, weil Elaiophylin und Elaiophylinderivate der Formel IV, in der $R^1$ Wasserstoff bedeutet, mit starken Basen wie Hydroxylionen, Metallalkoholaten oder Aminen in unkontrollierbarer Weise zersetzt werden können. Bei dem erfindungsgemäßen Verfahren geht man am besten so vor, daß man die genannten Ausgangsverbindungen mit einem bis zu 50-fachem Überschuß an einem Alkohol der Formel HO-$(CH_2)_n$-$R^4$ gegebenenfalls in Gegenwart katalytischer Mengen einer Lewissäure bis zur Beendigung der Reaktion umsetzt.

Eine Variante des Verfahrens besteht darin, daß man in einem geeigneten Lösungsmittel wie Chloroform,

Methylenchlorid, THF, Essigester oder Dioxan arbeitet. Als Lewissäure eignen sich beispielsweise Kupfer-, Eisen- oder Lithium-Halogenide, insbesondere $CuCl_2$, $FeCl_3$ oder LiBr.

Die Konzentration der Lewissäure - bezogen auf Elaiophylin oder das Elaiophylinderivat - beträgt 0,1 bis 5 Gew.-%, bevorzugt 0,5 bis 1 Gew.-%. Die Reaktionstemperaturen liegen dabei zwischen -40 °C und +100 °C, insbesondere zwischen 0 °C und 30 °C, vorzugsweise bei Verwendung eines Lösungsmittels zwischen dem Festpunkt und dem Siedepunkt des Lösungsmittels, insbesondere zwischen 0 °C und 30 °C. Die Reaktionszeiten betragen 1 bis 180 Minuten, bevorzugt 5 bis 60 Minuten. Die Beendigung der Reaktion kann beispielsweise mittels Dünnschichtchromatographie bestimmt werden.

Das als Ausgangssubstanz benötigte Elaiophylin kann beispielsweise nach dem in der deutschen Patentanmeldung P 37 21 722.4 vorgeschlagenen Verfahren hergestellt werden. Dabei fällt Elaiophylin als Fermentationsprodukt von Kulturen der Stämme Streptomyces violaceoniger DSM 4137 bzw. Streptomyces parvulus DSM 3816 an.

Zur Isolierung von Elaiophylin werden Kulturbrühe und Mycel, bevorzugt nur das Mycel, mit organischen Lösungsmitteln, wie Chloroform oder Essigester, bevorzugt Essigester, extrahiert.

Die Reinisolierung erfolgt durch Kristallisation aus dem organischen Lösungsmittel, bevorzugt aus Essigester.

Das als Ausgangssubstanz benötigte 2,2′,3,3′,4,4′,5,5′-Octahydroelaiophylin kann daraus nach dem Literaturverfahren von Kaiser et al. (Helv. Chim. Acta, 64 (1981), 407) hergestellt werden.

Mit Hilfe der Verfahrensvarianten b) lassen sich die C=C-Doppelbindungen des Macrodiolidringes hydrieren. Sofern das gewünschte Elaiophylinderivat ungesättigte oder reduzierbare Substituenten $R^1$ enthalten soll, erfolgt die Hydrierung des Macrodiolidringes zweckmäßigerweise vor der Ankoppelung des entsprechenden ungesättigten Substituenten nach Verfahren a).

Bei der Verfahrensvarianten b) geht man am besten so vor, daß man das zu hydrierende Elaiophylin oder Elaiophilinderivat, bevorzugt gelöst in einem Lösungsmittel wie Methanol, Ethanol, Isopropanol oder Ethylacetat oder einem Gemisch dieser Lösungsmittel in Gegenwart eines gängigen Hydrierkatalysators nach literaturbekannten Verfahren mit Wasserstoff umsetzt. Gängige Hydrierkatalysatoren sind beispielsweise Elemente der 8. Gruppe wie Platin, Palladium oder auch Nickel, die meist zum Zwecke der Vergrößerung der reaktiven Oberfläche, beispielswese auf Aktivkohle-, Siliziumdioxid- oder Aluminiumoxidträgern aufgetragen sind. Wird die Reaktion in einem absoluten primären Alkohol als Lösungsmittel durchgeführt, so erhält man mit speziellen Katalysatoren (siehe Beispiel 2) außer einer Hydrierung der C=C-Dippelbindungen auch eine Ketalisierung zu den $C_{11}/C_{11'}$-Di-O-Alkylen.

Je nach verwendetem Katalysator kann die Reaktion sowohl ohne als auch mit Überdruck an Wasserstoff, beispielsweise bis zu 1 Atmosphäre, durchgeführt werden. Die Reaktionstemperaturen liegen zwischen 0 °C und 40 °C, vorzugsweise bei Raumtemperatur. Die Reaktionszeiten sind abhängig von der Ansatzgröße und der Konzentration der zu reduzierenden Verbindung. Solche Hydrierungsverfahren sind beispielsweise beschrieben in Organikum, Organisch Chemisches Grundpraktikum, 15. Auflage, VEB Deutscher Verlag der Wissenschaften, Berlin, 1976, S. 359-371.

Mit Hilfe des Verfahrens c) wird das nach dem Verfahren a) oder b) hergestellte Elaiophylinderivat durch zweifache Umesterung des Macrodiolidringes mittels Alkoholaten in die beiden identischen seco-Elaiophylinester gespalten. Dabei geht man am besten so vor, daß man das Elaiophylinderivat mit einem 1,1 bis 5-fachen Überschuß des betreffenden Alkoholates $R^2O^-M^+$ in dem Alkohol $R^2OH$ als Lösungsmittel bis zur Beendigung der Reaktion umsetzt. Geeignet sind auch Lösungsmittelgemische aus dem Alkohol $R^2OH$ und einem geeigneten inerten Lösungsmittel wie Dichlormethan, Tetrahydrofuran oder Dioxan. Die Konzentration an Alkoholat $R^2O^-M^+$ kann im Bereich von 0,1-3,0 mol/l Lösungsmittel variiert werden, vorzugsweise arbeitet man mit einer Konzentration von 0,8 bis 1,2 mol/l. Die Reaktionstemperaturen liegen dabei zwischen -30 °C und +30 °C. Die Reaktionszeiten betragen 10 Minuten bis 6 Stunden, bevorzugt 30 Minuten bis 2 Stunden. Die Beendigung der Reaktion kann beispielsweise mittels Dünnschichtchromatographie an Kieselgel mit Lösungsmittelgemischen aus Dichlormethan und Methanol bestimmt werden.

Falls der mit Hilfe des Verfahrens a) eingeführte Rest $R^1$ mit dem Rest $R^2$ des Alkoholates $R^2O^-M^+$ übereinstimmt, so kann man die Verfahren a) und c) im Eintopfverfahren ohne Isolierung der nach Verfahren a) erhaltenen Zwischenprodukte durchführen. Dabei versetzt man die nach Verfahren a) erhaltene Produktlösung direkt mit der berechneten Menge des Alkoholates $R^2O^-M^+$ (0,1-3,0 mol/l), so daß die gewünschte Alkoholatkonzentration erreicht wird und verfährt dann wie oben beschrieben.

Mit Hilfe der Verfahrensvarianten e) und f) lassen sich die C=C-Doppelbindungen der seco-Elaiophylinderivate (I und III) hydrieren, wobei grundsätzlich analog Verfahren b) gearbeitet werden kann und aus Verbindungen I je nach Katalysatoraktivität und Rest $R^3$ entweder Produkte der allgemeinen Formeln (I) oder (III) entstehen. Seco-Elaiophylinderivate der allgemeinen Formel (I), in denen die beiden C=C-Doppelbindungen der aliphatischen Seitenkette intakt sind und die nach Verfahren h) acyliert wurden, können nur hydrierte seco-Elaiophylinderivate vom Typ (I) liefern, wobei alle gängigen, unter Verfahrensvariante b) aufgeführten Hydrierkatalysatoren geeignet sind. Dagegen reagieren die entsprechenden ungesättigten Derivate der Formel (I), in der $R^3$ Wasserstoff bedeutet, mit speziellen Hydrierkatalysatoren unter Eliminierung von $R^1OH$ zu den in der Seitenkette hydrierten Produkten (III). Ein Beispiel für einen solchen "speziellen Hydrierkatalysator" ist der Katalysator 10 proz. Palladium auf Kohle der Firma Merck. Dagegen erhält man aus den ungesättigten Derivaten der Formel (I), in der $R^3$ Wasserstoff bedeutet, z.B. mit dem Hydrierkatalysator 10 proz. Palladium auf Kohle der Firma Riedel-de-Häen die hydrierten Derivate der Formel (I). Die Produkte (III) entstehen als Stereoisomerengemisch bezüglich des neuen Asymmetriezentrums an C-11.

Das Verfahren g) erlaubt die Überführung der hydrierten seco-Elaiophylin-ester der Formeln (I) und (III), in denen X Sauerstoff, $R^1$ = H und $R^3$ Wasserstoff bedeutet, in die seco-Elaiophylin-säureamide durch Ersatz von $-OR^2$ gegen $-NH_2$ oder $NHR^2$.
Dabei geht man am besten so vor, daß man dem betreffenden seco-Elaiophylinester in äquimolaren Mengen oder in einem bis zu 100-fachen Überschuß mit dem Amin $H_2N-(CH_2)_n-R^4$ oder $NH_3$, gelöst in einem Lösungsmittel wie Methanol, Ethanol, Isopropanol, Ethylacetat, Tetrahydrofuran oder Dioxan, bis zur Beendigung der Reaktion umsetzt. Eine Variante besteht darin, daß man das Amin selbst als Lösungsmittel einsetzt.

Die Reaktionstemperaturen liegen dabei zwischen 20 °C und 100 °C, vorzugsweise bei Verwendung eines Lösungsmittels zwischen den Festpunkt und dem Siedepunkt des Lösungsmittel, insbesondere zwischen 20 °C und 60 °C. Die Reaktionszeiten betragen 1-72 Stunden, bevorzugt 8-48 Stunden. Die Beendigung der Reaktion kann beispielsweise mittels DC-kontrolle bestimmt werden.

Amine der Formel $H_2NR$ sind entweder käuflich erhältlich oder nach Standardverfahren der organischen Synthese leicht herzustellen.

Mit Hilfe der Verfahrensvariante h) lassen sich die Hydroxylgruppen in der 3′- und 4′-Stellung der seco-Elaiophylinderivate (I) und (III) Verestern. Geht man dabei von einer Verbindung der Formel (I) aus, in der $R^3$ Wasserstoff bedeutet, so wird noch zusätzlich selektiv die Hydroxylgruppe in der 7-Stellung verestert, während aus einer Verbindung der Formel (III), in der $R^3$ Wasserstoff bedeutet, zusätzlich noch die Hydroxylgruppe in der 9-Stellung verestert wird.
Daß in einer Verbindung der Formel (I) nur die Hydroxylgruppe in der 7-Stellung zusätzlich verestert wird und die Hydroxylgruppe in 9-Stellung erhalten bleibt, läßt sich aus dem experimentellen Befund schließen, daß ein Triacylderivat nach den Verfahrensvarianten d) und e) nur noch in ein Produkt des Strukturtyps (I) weiter umgesetzt werden kann, weil die Möglichkeit zur Cyclisierung n Derivate des Strukturtyps (III) blockiert ist. Bei der Verfahrensvarianten h) geht man am besten so vor, daß man das seco-Elaiophylinderivat der Formel (I) oder (III), in denen $R^3$ Wasserstoff bedeutet, in äquimolaren Mengen oder in einem bis zu 50-fachen Überschuß, gegebenenfalls in einem inerten, aprotischen Lösungsmittel wie Chloroform, Methylenchlorid, Tetrahydrofuran (THF), Essigester oder Dioxan, mit einer Verbindung der Formel V oder V′ bis zur Beendigung der Reaktion umsetzt, gegebenenfalls in Gegenwart einer Base, vorzugsweise Pyridin. Mit reaktionsträgen Reagenzien der Formel V oder V′ ist der Zusatz eines Acylierungskatalysators wie z.B. 4-Dimethylaminopyridin (DMAP) erforderlich.

Als nucleofuge Gruppen eignen sich Chlorid, Bromid, Imidazolid oder Säureanhydride.

Die Reaktionstemperaturen liegen dabei zwischen -70 °C und +100 °C, vorzugsweise bei Verwendung eines Lösungsmittels zwischen dem Festpunkt und dem Siedepunkt des Lösungsmittels, insbesondere zwischen -70 °C und +40 °C. Die Reaktionszeiten betragen 1 bis 180 Stunden, bevorzugt 1 bis 48 Stunden, besonders bevorzugt 1 bis 8 Stunden. Die Beendigung der Reaktion kann beispielsweise mittels Dünnschichtchromatographie bestimmt werden (DC-Kontrolle).

Die Ausgangsverbindungen für die Verfahrensvariante h), die Verbindungen der Formel V und/oder V′ sind, sofern nicht käuflich, auf einfache Weise nach literaturbekannten Verfahren herstellbar. Beispielsweise erhält man die Säurechloride (Z=U) durch Umsetzung der entsprechenden Carbonsäure mit Thionylchlorid, $PCl_3$ oder $PCl_5$. Solche Verfahren sind beispielsweise beschrieben in Gattermann/Wieland, "Die Praxis des Organischen Chemikers", 43. Auflage, Walter de Gruyter, Berlin, New York 1982, S. 303 ff.

Mit Hilfe des Verfahrens i) ist es möglich, die im Verfahrensschritt a) am Elaiophylin eingeführte Schutzgruppe $R^1$ in den seco-Elaiophylinderivaten der Formel (I) wieder abzuspalten und durch Wasserstoff zu ersetzen.

Dabei geht man so vor, daß man das entsprechende seco-Elaiophylinderivat der Formel (I), in der $R^1$ nicht Wasserstoff bedeutet, mit einer Lewissäure in einem wäßrigen sekundären oder tertiären Alkohol löst und bis zur Beendigung der Reaktion umsetzt. Die Konzentration der Lewissäure - bezogen auf das seco-Elaiophylinderivat - beträgt 0,1-5 Gew.-%, bevorzugt 0,5-1 Gew.-%. Als Lösungsmittel eignen sich z.B. Isopropanol, 2-Butanol oder tert.-Butanol, bevorzugt Isopropanol und der Wassergehalt beträgt 0,5-10 Gew.-% bevorzugt 0,5-2 Gew.-%. Die Reaktionstemperaturen liegen dabei zwischen -20°C und Raumtemperatur und die Reaktionszeiten liegen bei 5-60 Minuten.

Als Lewissäuren eignen sich die schon bei Verfahren a) aufgeführten Metallsalze, insbesondere $FeCl_3$.

Mit Hilfe der Verfahrensvariante d) lassen sich Verbindungen der allgemeinen Formel I, in der $R^3$ Wasserstoff bedeutet und in der die beiden C=C-Doppelbindungen auch hydriert sein können, in die Spiroverbindungen (III) überführen. Dabei geht man ganz analog dem Verfahren i) vor mit der Ausnahme, daß man die betreffende Verbindung der Formel (I) in einem wasserfreien Lösungsmittel umsetzt. Als Lösungsmittel eignen sich z.B. Dichlormethan, THF, Dioxan, Essigsäure-ethylester oder Alkohole wie Methanol, Ethanol, Propanol, Isopropanol, bevorzugt Methanol oder Ethanol.

Als Lewissäuren eignen sich die schon bei den Verfahren a) und i) aufgeführten Metallsalze, bevorzugt $FeCl_3$. Die Konzentration der Lewissäure, die Reaktionstemperatur und die Reaktionszeit liegen analog wie bei Verfahren i) beschrieben.

Die Reinigung, Isolierung und Aufarbeitung der Substanzen erfolgt nach den üblichen Methoden; beispielsweise können die Reaktionsprodukte durch Chromatographie an polaren Trägermaterialien wie Kieselgel oder ®Sephadex LH 20 mit Löusungsmitteln wie niederen Alkanolen wie Methanol oder Chloroform oder Essigester oder Methanol/Chloroform-Mischungen aber auch durch extraktive Methoden wie flüssig/flüssig-Extraktion oder fest/flüssig-Extraktion oder durch Kristallisation gereinigt werden.

Die Derivate des Elaiophylins zeigen anthelmintische Wirkung insbesondere gegen Haemonchus, Trichostrongylus Ostertagia, Cooperia, Chabertia, Strangyloides, Oesophagostomum, Hyostrongylus, Ancylostoma, Ascaris und Heterakis. Besonders ausgeprägt ist die Wirksamkeit gegenüber Magen-Darm-Strongyliden und Lungenwürmern, von denen vor allem Haus- und Nutztiere befallen werden.

Die Elaiophylinderivate können grundsätzlich als solche in Substanz verabreicht werden. Bevorzugt ist die Verwendung in Mischung mit geeignetem Trägermaterial. Als Trägermaterial können die üblichen Futtermittelmischungen verwendet werden.

In den sich anschließenden Beispielen wird die Erfirdung weiter erläutert. Prozentangaben beziehen sich auf das Gewicht und Mischungsverhältnisse bei Flüssigkeiten beziehen sich auf das Volumen, wenn keine anderen Angaben gemacht wurden.

Die Strukturaufklärung der neuen Verbindungen erfolgte mittels Elementaranalyse, Infrarot-Spektroskopie (IR), FAB-Massenspektrometrie (NaCl- oder KCl-Matrix) und zweidimensionalen (2D-) $^1$H- und $^{13}$C-Kernresonanzspektren.

Beispiel 1 (Verfahren a))

11,11′-Di-O-Methylelaiophylin (Formel IV, $R^1 = CH_3$)

20,0 g (19,5 mmol) Elaiophylin (Formel IV, $R^1$ = H) werden in 100 ml wasserfreiem Methanol suspendiert und mit 600 mg wasserfreiem Eisen(III)-chlorid versetzt. Rührt man diese Mischung ca. 2-5 Minuten bei 20 °C, so erhält man zunächst eine homogene orangegelbe Lösung. Nach Reiben mit einem Glasstab kristallisiert das Produkt spontan aus. Die Mutterlauge liefert nach Einengen und Kühlen noch weitere Produktfraktionen.
Ausbeute: 18,9 g (92 %)
Fp.: 171 °C
Analyse für $C_{56}H_{92}O_{18}$ (1053,3)
C (ber) 63,8 % H (ber) 8,8 %
C (gef) 63,6 % H (gef) 8,7 %

Beispiel 2 (Verfahren b))

2,2′,3,3′,4,4′,5,5′-Octahydro-11,11′-di-O-methyl-Elaiophylin (Formel IV, $R^1 = CH_3$, Macrodiolidring hydriert)

10,0 g (9,5 mmol) 11,11′-Di-O-Methylelaiophylin (aus Beispiel 1) werden in 100 ml Methanol suspendiert und mit 0,5 g 10 proz. Palladium auf Kohle (Fa. Riedel-de-Haen) bei Normaldruck und Raumtemperatur bis zur Aufnahme der berechneten Menge Wasserstoff hydriert. Nach Abfiltrieren des Katalysators und Einengen der Lösung zur Trockne kristallisiert man das Produkt aus Diisopropylether um.
Ausbeute: 9,78 (97 %)
Fp.: 114 °C (Zers.)
$[\alpha_D^{20}]$ - 49,5° (c = 1, $CH_3OH$)
C (ber) 63,3 % H (ber) 9,5 %
C (gef) 63,0 % H (gef) 9,7 %

Das Produkt kann auch in einem Schritt direkt aus Elaiophylin erhalten werden, wenn man in wasserfreiem

Methanol mit 10 proz. Palladium auf Kohle der Fa. Merck arbeitet (Ausbeute 87 %).

Beispiel 3 (Verfahren c)

11-O-Methyl-seco-Elaiophylin-methylester (Formel I, $R^1 = CH_3$, $XR^2 = OCH_3$)

2,15 g (2,04 mmol) 11,11′-Di-O-Methylelaiophylin (aus Beispiel 1) werden bei Raumtemperatur in 40 ml einer 1 M Natriummethanolat-Lösung gelöst. Nach 1,5 h gibt man auf einmal unter Rühren 5,35 g (100 mmol) Ammoniumchlorid zu und engt die Mischung am Rotationsverdampfer zur Trockne ein. Der Rückstand wird in Essigsäureethylester verrührt und das ausgefallene Kochsalz durch Filtration entfernt. Das nach Abdestillieren des Lösungsmittels zurückbleibende Öl wird mit Essigsäure-ethylester über Kieselgel filtriert. Das Produkt kann nach Einengen durch Verrühren mit n-Pentan als farbloses Pulver isoliert werden.
Ausbeute. 2,03 g (89 %)
($\alpha_D^{20}$] - 45° (c = 1, $CH_3OH$)
Analyse für $C_{29}H_{50}O_{10}$ (558,71)
C (ber) 62,3 % H (ber) 9,0 %
C (gef) 62,1 % H (gef) 9,1 %

Die Verbindung kann auch direkt aus Elaiophylin (Formel IV, $R^1$ = H) wie folgt hergestellt werden:

2,10 g (2,04 mmol) Elaiophylin werden in 20 ml wasserfreiem Methanol suspendiert und mit 60 mg wasserfreiem Eisen(III)chlorid versetzt. Nach 5 min Rühren bei Raumtemperatur gibt man 20 ml einer 2 M Natriummethanolat-Lösung hinzu und verfährt dann wie oben beschrieben.
Ausbeute: 1,80 g (78,9 %)

Beispiel 4 (Verfahren c)

2,3,4,5-Tetrahydro-11-O-methyl-seco-Elaiophylin-methylester (Formel I, hydriert, $R^1 = CH_3$, $XR^2 = OCH_3$)

2,00 g (1,88 mmol) 2,2′,3,3′,4,4′,5,5′-Octahydro-11,11′-di-O-methyl-Elaiphylin (aus Beispiel 2) werden in 30 ml einer 2 M Natriummethanolat-Lösung bei Raumtemperatur gelöst. Nach 5 h ist die Reaktion laut Dünnschichtchromatogramm (Kieselgel, Dichlormethan/Methanol 9/1, $R_f$ = 0,47) beendet. Nach Zugabe von 5,35 g (100 mmol) Ammoniumchlorid und 5 min Rühren engt man die Mischung am Rotationsverdampfer zur Trockne ein und arbeitet analog wie in Beispiel 3 beschrieben auf. Aus Diisopropylether kristallisieren 656 mg (31 %) Produkt vom Schmelzpunkt 81 °C.
FAB-MS (KCL-Matrix) Clusterion $(M+K)^+$: m/z = 601
Analyse für $C_{29}H_{54}O_{10}$ (562,74)
C (ber) 61,9 % (ber) 9,6 %
C (gef) 61,7 % (gef) 9,7 %

Beispiel 5 (Verfahren e)

2,3,4 5-Tetrahydro-11-O-methyl-seco-Elaiophylin-methylester (Formel I, hydriert, $R^1 = CH_3$, $XR^2 = OCH_3$)

2,60 g (4,65 mmol) 11-O-Methyl-seco-Elaiophylin-methylester (Produkt aus Beispiel 3) werden in 50 ml Methanol gelöst und mit 0,20 g 10 proz. Palladium/Kohle (Fa. Riedel-de-Haen) bei Normaldruck und Raumtemperatur bis zur Aufnahme der berechneten Menge Wasserstoff hydriert. Nach Abfiltrieren des Katalysators und Einengen wird der Rückstand aus Diethylether/Diisopropylether kristallisiert.
Ausbeute: 1,62 g (62 %)
Analytische Daten: siehe Beispiel 4)

Beispiel 6 (Verfahren g)

2,3,4,5-Tetrahydro-11-O-methyl-seco-Elaiophylincarboxamid (Formel I, hydriert, $R^1 = CH_3$, $XR^2 = NH_2$)

0,66 g (1,17 mmol) 2,3,4,5-Tetrahydro-11-O-methyl-seco-Elaiophylin-methylester (aus Beispiel 4 bzw. 5) werden in 50 ml einer bei 0 °C gesättigten Lösung von Ammoniak in Methanol gelöst und im verschlossenen Reaktionsgefäß 48 h bei Raumtemperatur belassen. Danach wird die Reaktionsmischung zur Trockne eingeengt und der Rückstand an Kieselgel chromatographiert. Mit Essigsäureethylester werden 100 mg 815 %)

der unumgesetzten Ausgangs-Verbindung zurückgewonnen. Beim Zusatz von 10 proz. Methanol in Essigsäureethylester wird das Produkt eluiert, das nach Einengen als farbloser Feststoff isoliert wird.
Ausbeute: 0,51g (79,5 %)
IR (KBr) 1675 $cm^{-1}$ (Amid-I), 1620 $cm^{-1}$ (Amid II)
[$\alpha_D^{20}$] -58,3° (c = 1, $CH_3OH$)
Analyse für $C_{28}H_{53}NO_9$ (547,7)
C (ber) 61,4 % H (ber) 9,7 % N (ber) 2,5 %
C (gef) 61,3 % H (gef) 9,6 % N (ber) 2,3 %

Beispiel 7 (Verfahren h)

3',4',7-Tri-O-Acetyl-11-O-Methyl-seco-Elaiophylin-methylester (Formel I, $R^1$ = $CH_3$, $XR^2$ = $OCH_3$, $R^3$ in 3'-,4'-,7-Position $COCH_3$)

1,12 g (2,01 mmol) 11-O-Methyl-seco-Elaiophylin-methylester (aus Beispiel 3) werden in 10 ml Dichlormethan, 10 ml Pyridin und 10 ml Acetanhydrid gelöst. Man läßt die Lösung 14 h bei Raumtemperatur stehen, verdünnt mit 200 ml Diethylether und schüttelt die organische Phase bis zur neutralen Reaktion mit gesättigter wäßriger Natirumhydrogencarbonat-Lösung aus. nach Trocknung über Natriumsulfat, Einengen und Filtration über Kieselgel mit Diisopropylether wird das Produkt aus Diisopropylether/n-Pentan umkristallisiert.
Ausbeute: 0,65 g (45 %)
Fp. 164-165 °C (Zers.)
IR (KBr) 1740 (Acetat), 1725 (Methylester), 1650/1620 (C=C)
[$\alpha_D^{20}$] -21,2° (c = 1, $CH_3OH$)
Charakteristische $^{13}C$-Daten (δ ppm in Pyridin-$d^5$):
93,90 (C-1'), 170,91, 170,76, 170,03 (OAc), 167,25 (C-1), 103,86 (C-11)
Analyse für $C_{35}H_{56}O_{13}$ (684,8)
C (ber) 61,4 % H (ber) 8,3 %
C (gef) 61,7 % H (gef) 8,5 %

Beispiel 8 (Verfahren e)

3',4',7(oder 9)-Tri-O-Acetyl-11-O-Methyl-2,3,4,5-Tetrahydro-seco-Elaiophylin-methylester (Formel I, hydriert, $R^1$ = $CH_3$, $XR^2$ = $OCH_3$, $R^3$ in 3'-,4'-,7-Position $COCH_3$)

0,80 g (1,16 mmol) 3',4',7-Tri-O-Acetyl-11-O-Methyl-seco-Elaiophylin-methylester (aus Beispiel 7) werden in 15 ml Methanol gelöst und mit 0,10 g 10 proz. Palladium/Kohle (Fa. Riedel-de-Haen) bei Normaldruck und Raumtemperatur bis zur Aufnahme der berechneten Menge Wasserstoff hydriert. Nach Filtration über Corolite, Einengen und Trocknung im Vakuum erhält man 0,79 g (99 %) Produkt als farblosen Feststoff.
IR (KBr) 1740 (OAc), 1720 (Methylester)
[$\alpha_D^{20}$] -49° (c = 1, $CH_3OH$)
FAB-MS (KCL-Matrix) Clusterion $(M+K)^+$: m/z = 727
Analyse für $C_{35}H_{60}O_{13}$ (688,85)
C (ber) 61,0 % H (ber) 8,8 %
C (gef) 61,4 % H (gef) 8,2 %
Charakteristische $^{13}C$-Daten (δ, ppm in Pyridin-$d^5$)
173,76 (C-1), 171,66, 170,79, 170,04 (OAc), 103,86 (C-11), 93,86 (C-1').

Beispiel 9 (Verfahren h)

Das in Beispiel 8 beschriebene Produkt läßt sich auch noch wie folgt herstellen:
0,56 g (1,00 mmol) 2,3,4,5-Tetrahydro-11-O-methyl-seco-Elaiophylin-methylester (aus Beispiel 5) werden in 2 ml Dichlormethan, 2 ml Pyridin und 2 ml Acetanhydrid gelöst. Man läßt die Lösung 16 h bei Raumtemperatur stehen und arbeitet analog wie in Beispiel 7 beschrieben auf.
Ausbeute: 0 48 g (69 %)
Analytische Daten: siehe unter Beispiel 8.

Beispiel 10 (Verfahren i)

3′,4′,7-Tri-O-Acetyl-seco-Elaiophylin-methylester (Formel I, $R^1$ = H, $XR^2$ = $OCH_3$, $R^3$ in 3′-,4′-,7-Position $COCH_3$)

1,37 g (2,00 mmol) 3′,4′,7-Tri-O-Acetyl-11-O-Methyl-seco-Elaiophylin-methylester (aus Beispiel 7) werden in 10 ml Isopropanol und 0,1 ml Wasser gelöst. Man versetzt mit 50 mg Kupfer(II)-chlorid und rührt bei Raumtemperatur bis zur Beendigung der Reaktion (DC Kontrolle an Kieselgel mit Dichlormethan/Methanol 40/1) (Reaktionszeit ca. 15 min).

Nach Zugabe von 100 ml Ether wird die organische Phase zweimal mit je 50 ml Natriumhydrogencarbonat-Lösung gewaschen, getrocknet ($Na_2SO_4$) und eingeengt. Das Produkt wird aus Diisopropylether/n-Pentan kristallisiert.
Ausbeute: 0,79 g (58,9 %)
Fp.: 147-148 °C (Zers.)
[$\alpha_D^{20}$] -59° (c=1, $CH_3OH$)
FAB-MS (KCl-Matrix) Clusterion $(M+K)^+$ m/z = 709
Analyse für $C_{34}H_{54}O_{13}$ (670,8)
C (ber) 60,9 % H (ber) 8,1 %
C (gef) 61,2 % H (gef) 8,2 %
Charakteristische $^{13}C$-Daten (δ ppm in Pyridin-$d^5$):
171,10, 170,81, 170,06 (OAc), 167,25 (C-1), 100,08 (C-11), 93,81 (C-1′).

Beispiel 11 (Verfahren e)

3′,4′,7-Tri-O-Acetyl 2,3,4,5-Tetrahydro-seco-Elaiophylin-methylester (Formel I, hydriert, $R^1$ = H, $XR^2$ = $OCH_3$, $R^3$ in 3′-,4′-,7-Position $COCH_3$)

0,45 g (0,67 mmol) Produkt aus Beispiel 10 werden in 10 ml Essigsäure-ethylester gelöst und mit 100 mg 10 proz. Palladium/Kohle (Fa. Riedel-de-Haen) bei Normaldurck und Raumtemperatur bis zur Aufnahme der berechneten Menge Wasserstoff hydriert. Nach Abfiltrieren des Katalysators und Abziehen des Lösungsmittels wird der Rückstand aus Diisopropylether/n-Pentan kristallisiert.
Ausbeute: 0,36 g (79 %)
Fp.: 112-113 °C
Analyse für $C_{34}H_{58}O_{13}$ (674,8)
C (ber) 60,5 % H (ber) 8,6%
C (gef) 60,3 % H (gef) 8,6 %
$^{13}C$-NMR-Daten (δ ppm in Pyridin-$d^5$)
173,78 (C-1),
171,88, 170,84, 170,10 (OAc)
100,04 (C-11)
93,85 (C-1′)
77,40 (C-7)
70,93 (C-9)
70,82 (C-13)
70,34 (C-4′)
67,53 (C-3′)
66,88 (C-15)
65,55 (C-5′)
51,25 ($CO_2CH_3$)
48,90(C-14)
43,11 (C-8)
38,93 (C-12)
38,29 (C-10)
34,99, 32,91 (C-2, C-3)
34,09 (C-6)
31,00 (C-2′)
26,41, 25,52 (C-4, C-5)

20,86, 20,75, 20,56 (OAc)
19,77 (C-20)
19,51, 16,84, 15,73, 9,74, 9,11, 7,46 ($CH_3$)

Beispiel 12 (Verfahren h)

3′,4′,7(oder 9)-Tri-O-Benzoyl-11-O-methyl-seco-Elaiophylin-methylester (Formel I, $R^1 = CH_3$, $XR^2 = OCH_3$, $R^3$ in 3′-,4′-, 7-Position $COC_6H_5$)

1,08 g (1,93 mmol) 11-O-Methyl-seco-Elaiophylin-methylester (aus Beispiel 3), 2,26 g (10,0 mmol) Benzoesäureanhydrid und 300 mg (2,45 mmol) 4-Dimethylaminopyridin werden in 15 ml Dichlormethan und 15 ml Pyridin gelöst. Nach 5 h bei Raumtemperatur gibt man 3 ml wasserfreies Methanol hinzu und arbeitet nach 10 min wie folgt auf: Nach Verdünnen mit 150 ml Diethylether wird die organische Phase 3 mal mit je ml einer gesättigten wäßrigen natriumhydrogencarbonat-Lösung gewaschen, getrocknet ($Na_2SO_4$) und eingeengt. Der Rückstand wird an Kieselgel mit Essigsäure-ethylester/Hexan (1/2) chromatographiert. Das Produkt ist ein farbloser Feststoff.
Ausbeute: 0,85 g (50,5 %)
$[\alpha_D^{20}]$ -37° (c = 1, $CH_3OH$)
C (ber) 68,9 % H (ber) 7,1 %
C (gef) 68,6 % H (gef) 7,1 %
IR (KBr)
1690 (Methylester)
1725 (Benzoat)

Beispiel 13 (Verfahren e)

7,11-Anhydro-2,3,4,5-tetrahydro-seco-Elaiophylin-methylester (Formel III, $XR^2 = OCH_3$, $R^3 = H$)

1,25 g (2 23 mmol) 11-O-Methyl-seco-Elaiophylin-methylester (aus Beispiel 3) werden in 50 ml wasserfreiem Methanol gelöst und mit 300 mg 10 proz. Palladium/Kohle (Fa. Merck) bei Raumtemperatur und Normaldruck bis zur Aufnahme der berechneten Menge Wasserstoff hydriert. Die Reaktionslösung wird eingeengt und der Rückstand an 200 g Kieselgel mit Essigsäureethylester/Hexan (2/1) chromatographiert. Die im folgenden mit (A) und (B) bezeichneten stereoisomeren Produkte werden dabei glatt getrennt und als farblose Öle in einer Gesamtausbeute von 82 % erhalten.

Produkt (A)

Ausbeute: 0,55 g (47 %)
$R_f$-Wert: 0,30 (an Kieselgel mit $CH_2Cl_2/CH_3OH$ 15/1)
$[\alpha_D^{20}]$ -119° (c = 1, $CH_3OH$)
IR ($CH_2Cl_2$) 1720 $cm^{-1}$ (Methylester)
Analyse für $C_{28}H_{50}O_9$ (530,69)
C (ber) 63,3 % H (ber) 9,5 %
C (gef) 63,0 % H (gef) 9,4 %
FAB-MS (NaCl-Matrix) Clusterion $(M + Na)^+$ m/z = 553
Ausgewählte $^{13}C$-Daten (δ, ppm in Pyridin-$d^5$)
173,95 (C-1), 100,74 (C-11), 94,56 (C-1′), 51,18 (Ester-$CH_3$)

Produkt (B)

Ausbeute: 0,41 g (35 %)
$R_f$-Wert: 0,19 (an Kieselgel mit $CH_2Cl_2/CH_3OH$ 15/1)
$[\alpha_D^{20}]$ -87° (c = 1, $CH_3OH$)
IR ($CH_2Cl_2$) 1720 $cm^{-1}$ (Methylester)
Analyse für $C_{28}H_{50}O_9$ (530,69)
C (ber) 63,3 % H (ber) 9,5 %
C (gef) 63,1 % H (gef) 9,6 %

FAB-MS (NaCl-Matrix) Clusterion $(M + Na)^+$ m/z = 553
Ausgewählt $^{13}C$-Daten (δ, ppm in Pyridin-$d^5$)
173,82 (C-1), 102,03 (C-11), 94,34 (C-1′), 51,18 (Ester-$CH_3$)

Das Produkt aus Beispiel 4 ist dünnschichtchromatographisch nicht nachweisbar.

Beispiel 14 (Verfahren h)

7,11-Anhydro-3′,4′,9-tri-O-acetyl-2,3,4,5-tetrahydro-seco-Elaiophylin-methylester Stereoisomer (A) (Formel III, $XR^2 = OCH_3$, $R^3 = COCH_3$)

1,10 g (2,07 mmol, 7,11-Anhydro-2,3,4,5-tetrahydro-seco-Elaiophylin-methylester (Stereoisomer A aus Beispiel 13) werden in 7 ml Dichlormethan, 7 ml Pyridin und 7 ml Acetanhydrid 40 h bei Raumtemperatur acetyliert. Nach Verdünnung mit 200 ml Diethylether wird die organische Phase mit gesättigter wäßriger Natriumhydrogencarbonat-Lösung bis zur neutralen Reaktion gewaschen. Trocknung ($Na_2SO_4$), Einengen und Säulenfiltration über Kieselgel mit Diisopropylether/Hexan (1/1) liefert das Produkt als farbloses, viskoses Öl.
Ausbeute: 1,20 g (88 %)
$[\alpha_D^{20}]$ -133° (c = 1, $CH_3OH$)
FAB-MS (KCl-Matrix) Clusterion $(M + K)^+$ m/z = 695
Analyse für $C_{34}H_{56}O_{12}$ (656,81)
C (ber) 62,1 % H (ber) 8,6 %
C (gef) 61,9 % H (gef) 8,4 %
Ausgewählte $^{13}C$-Daten (δ, ppm in Pyridin-$d^5$)
173,90 (C-1), 170,79, 170,34, 169,94 (OAc), 100, 53 (C-11), 94,42 (C-1′), 51,24 (Ester-$CH_3$)

Beispiel 15 (Verfahren h)

7,11-Anhydro-3′,4′,9-tri-O-acetyl-2,3,4,5-tetrahydro-seco-Elaiophylin-methylester, Stereoisomer (B) (Formel III, $XR^2 = OCH_3$, $R^3 = COCH_3$)

0,70 g (1,32 mmol) 7,11-Anhydro-2,3,4,5-tetrahydro-seco-Elaiophylin-methylester (Stereoisomer B aus Beispiel 13) werden in 5 ml Dichlormethan, 5 ml Pyridin und 5 ml Acetanhydrid 40 h bei Raumtemperatur acetyliert. Die Isolierung des Produktes erfolgt ganz analog wie in Beispiel 14 beschrieben.
Ausbeute: 0,85 g (98 %) als farbloser Feststoff
FAB-MS (KCl-Matrix) Clusterion $(M + K)^+$ m/z 695
Analyse für $C_{34}H_{56}O_{12}$ (656,81)
C (ber) 62,1 % H (ber) 8,6 %
C (gef) 61,8 % H (gef) 8,3 %
Ausgewählte $^{13}C$-Daten (δ, ppm in Pyridin-$d^5$)
173,76 (C-1), 170,77, 170,34, 170,10 (OAc), 101,80 (C-11), 94,04 (C-1′), 51,25 (Ester-$CH_3$)

Beispiel 16 (Verfahren d)

Darstellung der Produkte A und B des Beispiels 13 nach Verfahren d)

563 mg (1,00 mmol) 2,3,4,5-Tetrahydro-11-O-methyl-seco-Elaiophylin-methylester (Formel I, hydriert, $R^1 = CH_3$, $XR^2 = OCH_3$) aus Beispiel 4 oder aus Beispiel 5 werden in 5 ml Methanol (wasserfrei) gelöst und bei Raumtemperatur mit 10 mg $FeCl_3$ versetzt. Nach 10 min engt man ein und trennt die Produkte wie in Beispiel 13 beschrieben.
Ausbeute Produkt A: 239 mg (45 %)
Ausbeute Produkt B: 212 mg (40 %)
Analytische Daten siehe unter Beispiel 13.

Beispiel 17 (Verfahren d)

7,11-Anhydro-seco-Elaiophylin-methylester (Formel III, $XR^2 = OCH_3$, $R^3 = H$)

0,95 g (1,70 mmol) 11-O-methyl-seco-Elaiophylin-methylester aus Beispiel 3 werden bei 20 °C in 10 ml

wasserfreiem Methanol gelöst und mit 30 mg $FeCl_3$ versetzt. Nach 10 min engt man ein und chromatographiert an 50 g Kieselgel mit Ethylacetat/Hexan (2/1). Dabei werden die C-11-epimeren Produkte A und B glatt getrennt und nach Trocknung im Hochvakuum als farblose Feststoffe isoliert.

C-11-Epimer (A):

Ausbeute 0,44 g (49 %)
$R_f$-Wert: 0,30 (an Kieselgel mit Echylacetat)
$[\alpha_D^{20}]$ -134° (c = 1, $CH_3OH$)
IR (KBr) 1715 (c = 0), 1640, 1620 $cm^{-1}$ (C=C)
FAB-MS (NaCl-Matrix) Clusterion $(M + Na)^+$
m/z = 549
Analyse für $C_{28}H_{46}O_9$ (526,66)
C (ber) 63,8 % H (ber) 8,8 %
C (gef) 63,5 % H (gef) 8,7 %

C-11-Epimer (B):

Ausbeute: 0,38 g (42 %)
$R_f$-Wert: 0,16 (an Kieselgel mit Ethylacetat)
$[\alpha_D^{20}]$ -58,2° (c = 1, $CH_3OH$)
IR (KBr) 1715 (c = 0), 1640, 1620 $cm^{-1}$ (C=C)
FAB-MS (NaCl-Matrix) Clusterion $(M + Na)^+$ m/z = 549
Analyse für $C_{28}H_{46}O_9$ (526,66)
C (ber) 63,8 % H (ber) 8,8 %
C (gef) 63,6 % H (gef) 8,8 %

Beispiel 18 (Verfahren a) und c))

11-O-(2-Phenylethyl)-seco-Elaiophylin-methylester (Formel I, $R^1 = CH_2CH_2C_6H_5$, $XR^2 = OCH_3$)

2,00 g (1,95 mmol) Elaiophylin (Formel IV, $R^1$ = H) werden in einer Lösung von 3,00 g (24,6 mmol) 2-Phenylethanol in 17 ml Dichlormethan suspendiert und mit 100 mg wasserfreiem Eisen(III)chlorid versetzt. Nach ca. 5 min. Rühren bei 20 °C erhält man eine homogene Lösung und die Ausgangsverbindung hat sich quantitativ umgesetzt (DC-Kontrolle: Dichlormethan/Methanol 9/1). Man gibt nun 40 ml einer 1M Natriummethanolat-Lösung hinzu und rührt 1,5 h bei Raumtemperatur. Nach 1,5 h versetzt man mit 5,35 g (100 mmol) Ammoniumchlorid und engt die Mischung am Rotationsverdampfer zur Trockne ein. Die weitere Aufarbeitung erfolgt analog wie in Beispiel 3 beschrieben.
Ausbeute: 0,94 g (37 %)
Analyse für $C_{36}H_{56}O_{10}$ (648,83)
C (ber) 66,6 % H (ber) 8,69 %
C (gef) 66,4 % H (gef) 8,72 %
IR ($CHCl_3$) 1695 $cm^{-1}$ (Ester)

Beispiel 19 (Verfahren a) und c))

11-O-Propyl-seco-Elaiophylin-methylester (Formel I, $R^1 = C_3H_7$, $XR^2 = OCH_3$)

2,00 g (1,95 mmol) Elaiophylin (Formel IV, $R^1$ = H) werden in einer Lösung von 1,2 g (20 mmol) n-Propanol in 18 ml Dichlormethan suspendiert und mit 100 mg wasserfreiem Eisen(III)chlorid versetzt. Nach ca. 5 min. Rühren bei 20 °C erhält man eine homogene Lösung (DC-Kontrolle: analog Beispiel 16). Nach Zugabe von 40 ml einer 1 M Natriummethanolat-Lösung und 1,5 stündigem Rühren bei Raumtemperatur setzt man 5,35 g (100 ml) Ammoniumchlorid zu und isoliert das Produkt analog wie in Beispiel 3 beschrieben.
Ausbeute 1,20 g (52 %)
Analyse für $C_{31}H_{54}O_{10}$ (586,76)
C (ber) 63,4 % H (ber) 9,2 %
C (gef) 63,1 % H (gef) 9,0 %

IR ($CHCl_3$) 1695 $cm^{-1}$ (Ester)

Anthelminthische Wirkung der Elaiophylinderivate

Die anthelminthische Wirkung der Elaiophylinderivate wurde an Lämmern mit 30 bis 40 kg Körpergewicht untersucht. Dazu wurden die Lämmer artifiziell mit Infektionsstadien von Labmagennematoden (Haemunchus contortus) infiziert. Nach Abschluß der Entwicklungszeit (Präpatenzperiode) der Nematoden erfolgte die Applikation der Elaiophylinderivate.

Durch koproskopische Untersuchungen vor und bis zu 14 Tagen nach der Applikation der Elaiophylinderivate und anschließender Sektion mit helminthologischer Aufarbeitung wurde die prozentuale Reduktion der Schaf-Nematoden ermittelt (s. Tabelle 1). Als Vergleichssubstanz diente Elaiophylin. Zusätzlich wurde auch noch die antibakterielle Aktivität der erfindungsgemäßen Elaiophylinderivate gegen Staph. aureus und Strept. pyogenes ermittelt. Überraschenderweise zeigen die erfindungsgemäßen Verbindungen keine oder nur sehr geringe antibakterielle Aktivität. Gerade deshalb eignen sich die erfindungsgemäßen Verbindungen insbesondere für den Einsatz als Anthelminthika, da hier eine antibakterielle Wirkung unerwünscht ist.

**Tabelle 1**

| Verabreichte Verbindung aus Beispiel | Dosierung (mg/kg) | Reduktion v. H. contortus (%) | antibakterielle Aktivität (µg/ml) gegen Stap. aureurs | Strep. pyogenes |
|---|---|---|---|---|
| Elaiophylin | 2,5 (s.c.) | 35-45 | 1,56 | 1,56 |
| | 5,0 (oral) | 70-95 | | |
| 3 | 2,5 (s.c.) | - | >100 | >100 |
| | 5,0 (oral) | 70-90 | | |
| 4 | 2,5 (s.c.) | 50-70 | >100 | >100 |
| | 5,0 (oral) | 30-40 | | |
| 7 | 2,5 (s.c.) | 40-75 | >100 | >100 |
| | 5,0 (oral) | 50-70 | | |
| 10 | 2,5 (s.c.) | 30-70 | >100 | >100 |
| | 5,0 (oral) | - | | |
| 17 | 2,5 (s.c.) | - | >100 | >100 |
| | 5,0 (oral) | 30-40 | | |

Reaktionsschema

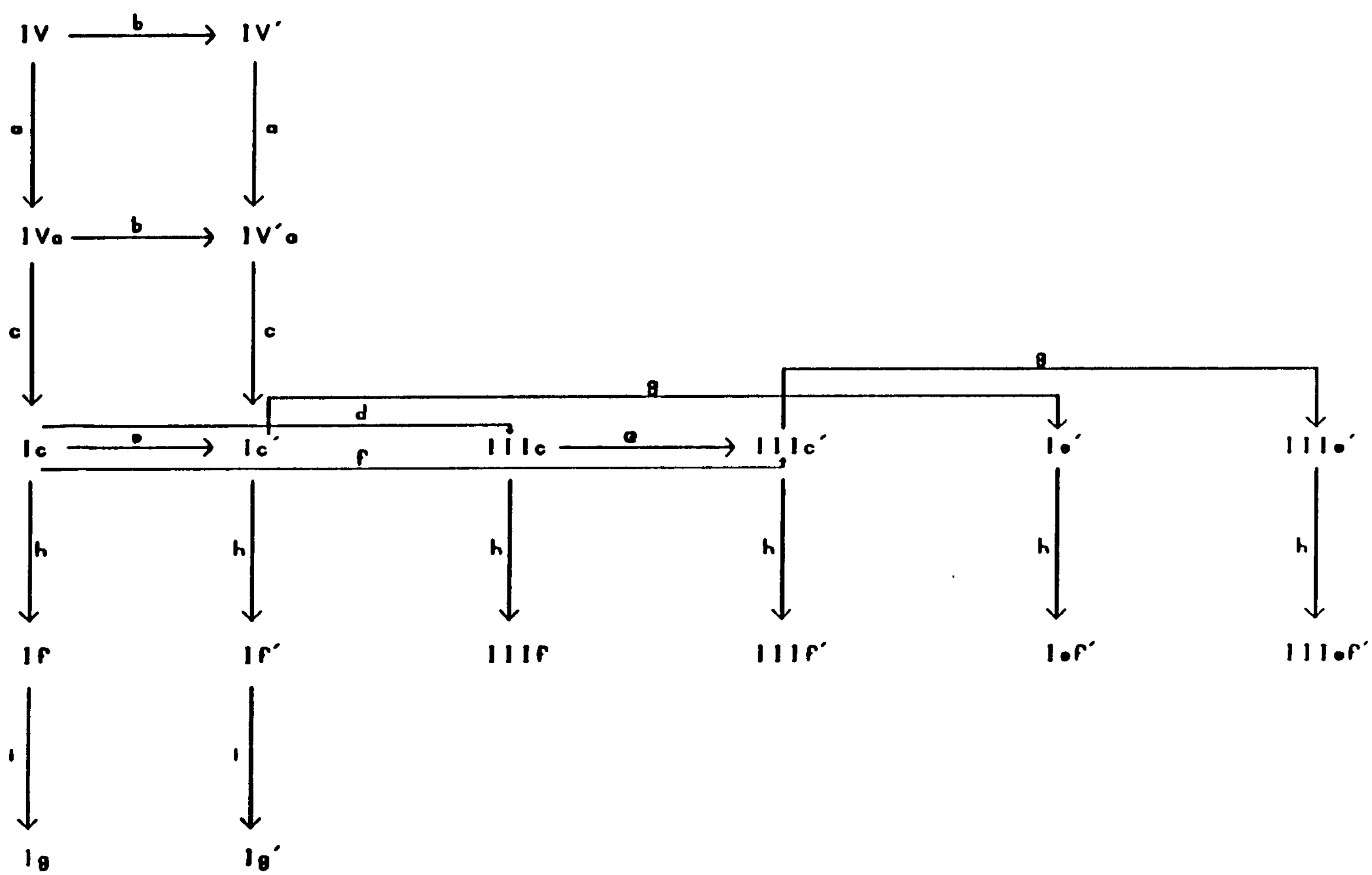

| Verbindung | Generelle Formel | $R^1$ | X | $R^2$ | $R^3$ |
|---|---|---|---|---|---|
| IV | IV | H | - | - | - |
| IV' | IV' | H | - | - | - |
| IVa | IV | R | - | - | - |
| IVa' | IV' | R | - | - | - |
| Ic | I | R | O | R | H |
| Ic' | I' | R | O | R | H |
| IIIc | III | - | O | R | H |
| IIIc' | III' | - | O | R | H |
| Ie' | I' | R | NH | R bzw. H | H |
| IIIe' | III' | - | NH | R bzw. H | H |
| If | I | R | O | R | CO-R |
| If' | I' | R | O | R | CO-R |
| IIIf | III | - | O | R | CO-R |
| IIIf' | III' | - | O | R | CO-R |
| Ief' | I' | R | NH | R bzw. H | CO-R |
| IIIef' | III' | - | NH | R bzw. H | CO-R |
| Ig | I | H | O | R | CO-R |
| Ig' | I' | H | O | R | CO-R |

R bedeutet $(CH_2)_n$-$R^4$ n und $R^4$ die in Anspruch 1 angegebenen Bedeutungen haben.

## Patentansprüche

1. Elaiophylinderivate der Formeln (I) und (III)

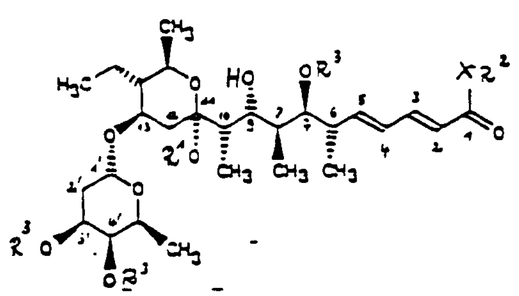

I

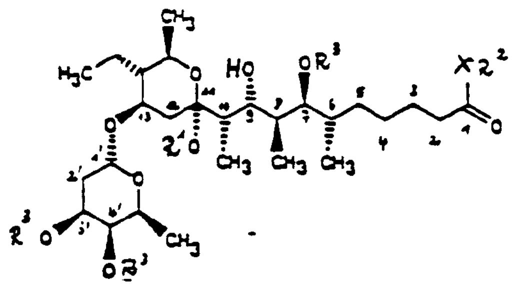

I

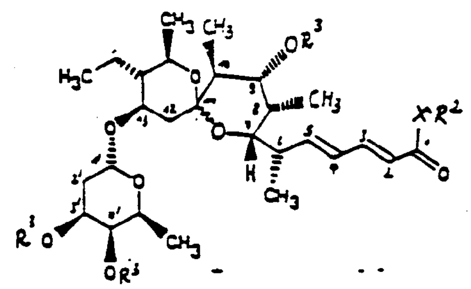

III

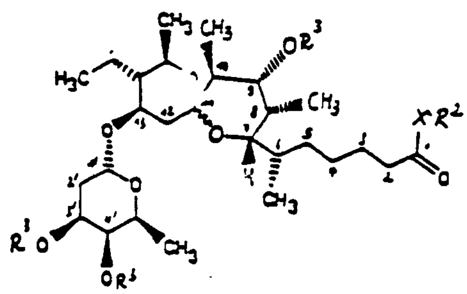

III'

worin die C=C Doppelbindungen in 2,3 und 4,5 Position auch hydriert sein können (Formeln (I') und (III') und in denen

$R^1$ Wasserstoff oder ein Rest der Formel (VI)

$$-(CH_2)_n-R^4 \qquad (VI)$$

bedeutet,

wobei

n = 1 bis 3 und

$R^4$ Wasserstoff, $C_1$-$C_{15}$-Alkyl, $C_2$-$C_{15}$-Alkenyl, $C_2$-$C_{15}$-Alkinyl, $C_3$-$C_9$-Cycloalkyl, Phenyl oder ein Heteroaryl mit 3 bis 9 Ringatomen ist, wobei die Ringverbindungen gegebenenfalls Chlor, Brom, Jod, nitro-, hydroxy- oder $C_1$-$C_4$-alkyl- oder $C_1$-$C_4$-alkoxy-substituiert sind,

X 0 oder, wenn $R^1$ nicht Wasserstoff ist und die C=C Doppelbindungen in Formel (I) und (III) in 2,3 und 4,5 Position hydriert sind, auch NH bedeutet,

$R^2$ Wasserstoff oder $C_1$-$C_4$-Alkyl bedeutet, oder, falls X NH ist, ein Rest der Formel VI darstellt, wobein und $R^4$ die oben zu Formel VI angegebenen Bedeutungen haben und worin

$R^3$ Wasserstoff oder ein Rest der Formel (II)

$$-\overset{\overset{\displaystyle O}{\|}}{C}-(CH_2)_n-R^4 \qquad (II)$$

bedeutet worin n und $R^4$ die oben zu Formel VI angegebenen Bedeutungen haben.

2. Elaiophylinderivate der Formeln (I) und (III) nach Anspruch 1, in denen $R^4$ Wasserstoff, $C_1$-$C_6$-Alkyl, Phenyl oder Naphtyl, Thiophen, Furan, Pyridin, Pyrimidin oder Pyrazin ist.

3. Elaiophylinderivate der Formeln (I) und (III) nach Anspruch 1 und/oder 2 in denen $R^4$ Wasserstoff, $C_1$-$C_4$-Alkyl oder Phenyl ist.

4. Verfahren zur Herstellung von Elaiophylinderivaten der Formel I und III, dadurch gekennzeichnet, daß man

a) Elaiophylin der Formel IV,

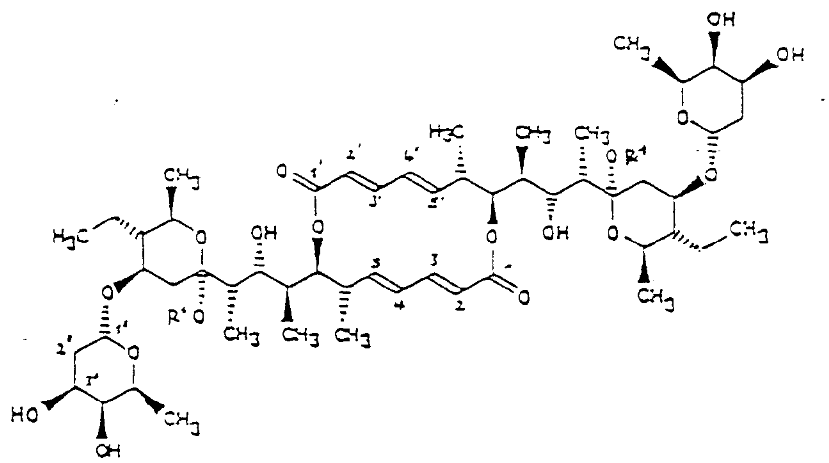

(IV)

wobei $R^1$ Wasserstoff bedeutet, mit einem Alkohol der Formel $HO-(CH_2)_n-R^4$, worin n und $R^4$ die in Anspruch 1 angegebene Bedeutung haben, umsetzt zu einer Verbindung der Formel IV, in der $R^1$ $(CH_2)_n-R^4$ bedeutet, wobei n und $R^4$ die in Anspruch 1 angegebenen Bedeutungen haben und das so erhaltene Zwischenprodukt, gegebenenfalls nach seiner Isolierung entweder zunächst

b) in Gegenwart von Hydrierkatalysatoren zu der Verbindung der Formel IV′ reduziert,

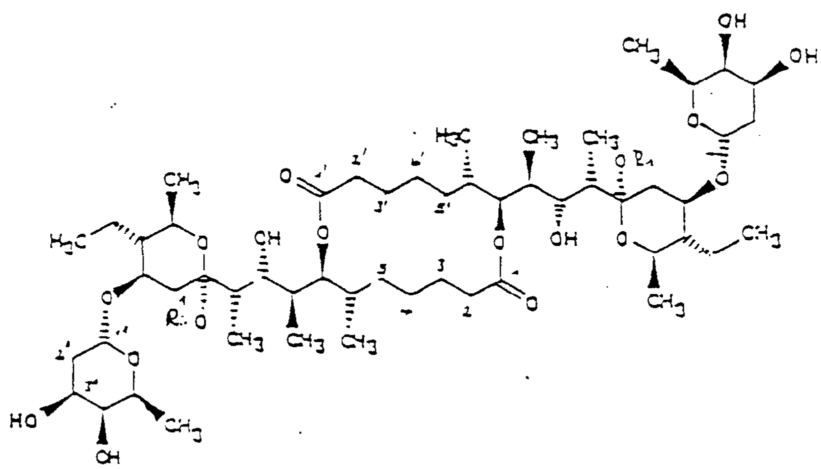

(IV')

in der die C=C Doppelbindungen in Position 2, 3 und 4, 5 sowie 2′, 3′ und 4′, 5′ hydriert sind und $R^1$ die in a) angegebene Bedeutung hat, oder

c) das nach Reaktion a) erhältliche Zwischenprodukt oder das nach Reaktion b) erhältliche Zwischenprodukt mit Alkalimetallalkoholaten der Formel $R^2O^-M^+$, worin M Lithium, Natrium oder Kalium bedeuten und $R^2$ die in Anspruch 1 angegebeneBedeutung hat, umsetzt zu Verbindungen der Formel I oder I′, worin $R^3$ Wasserstoff, $XR^2$ einen Rest $OR^2$ und $R^1$ und $R^2$ außer Wasserstoff die in Anspruch 1 angegebenen Bedeutungen haben, oder

d) eine Verbindung erhalten nach c) in einem nichtwäßrigen Lösungsmittel umsetzt mit einer LEWIS-Säure, zu einer Verbindung der Formel III, in der $XR^2$ und $R^3$ die in c) gegebene Bedeutung haben, oder

e) eine Verbindung erhalten nach c) oder d) mit Wasserstoff in Gegenwart von Hydrierkatalysatoren zu Verbindungen der Formeln I′ oder III′ reduziert, worin $R^1$, $XR^2$ und $R^3$ die in c) oder d) gegebene Bedeutung haben, oder

f) eine Verbindung erhalten nach c) mit Hydrierkatalysatoren, die zusätzlich eine LEWIS-Säure Aktivität aufweisen zu einer Verbindung erhalten nach e) reduziert, oder

g) eine Verbindung erhalten nach c) oder d) umsetzt mit Ammoniak oder einem primären Amin der Formel $H_2N$-$(CH_2)_n$-$R^4$, in der n und $R^4$ die in Anspruch 1 angegebenen Bedeutungen haben, zu Verbindungen der Formel I′ oder III′, in denen X NH bedeutet, oder

h) eine Verbindung erhalten nach c), d) oder g) umsetzt mit einer Verbindung der Formel Z-CO-$(CH_2)$-$R^4$, worin n und $R^4$ die in Anspruch 1 angegebene Bedeutung haben und in der Z eine nucleofuge Gruppe bedeutet, zu einer Verbindung der Formeln (I) oder (III) bzw, (I′) oder (III′), worin $R^1$, $XR^2$ und $R^3$ die in Anspruch 1 angegebenen Bedeutungen haben, oder

i) eine Verbindung der Formel (I) oder (I′), worin $R^1$ und $R^2$, bis auf Wasserstoff, sowie $R^3$ die in Anspruch 1 angegebene Bedeutung haben, in Gegenwart eines wäßrigen Lösungsmittels mit einer LEWIS-Säure umsetzt, wobei man eine Verbindung der Formel (I) oder (I′) erhält, in der $R^1$ Wasserstoff bedeutet und $XR^2$ und $R^3$ gegenüber der Ausgangsverbindung (I) oder (I′) für die Reaktion i) unverändert bleiben.

5. Verfahren gemäß Anspruch 4, dadurch gekennzeichnet, daß zunächst Elaiophylin (Formel IV, $R^1 = R^2 = R^3 = H$) gemäß Reaktion b) aus Anspruch 4 zu der Verbindung (IV′) hydriert wird, dann gemäß Reaktion a) mit einem Alkohol der Formel HO-$(CH_2)_n$-$R^4$, worin n und $R^4$ die in Anspruch 1 angegebenen Bedeutungen haben, zu einer Verbindung der Formel (IVa′) umgesetzt wird.

6. Elaiophylinderivate gemäß Anspruch 1 zur Verwendung als Arzneimittel.

7. Elaiophylinderivate gemäß Anspruch 1 zur Verwendung als Anthelmintikum.

8. Arzneimittel mit anthelmintischer Wirkung enthaltend anthelmintisch wirkende Menge einer oder mehrerer verschiedener Verbindungen der Formeln (I) und/oder (III) gemäß Anspruch 1 und gegebenenfalls pharmakologisch verträgliche Hilfsstoffe oder/und Trägerstoffe und gegebenenfalls andere Wirkstoffe.

## Claims

1. An elaiophylin derivative of the formula (I) or (III)

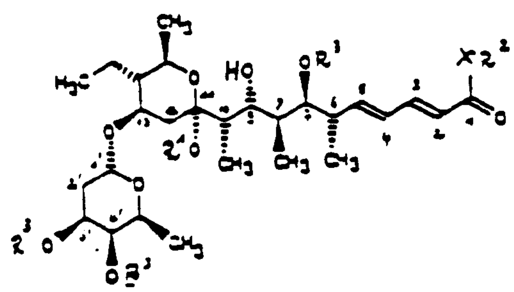

I

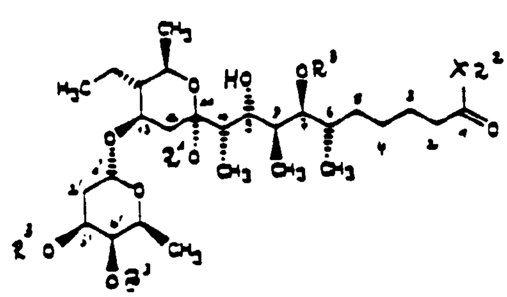

I''

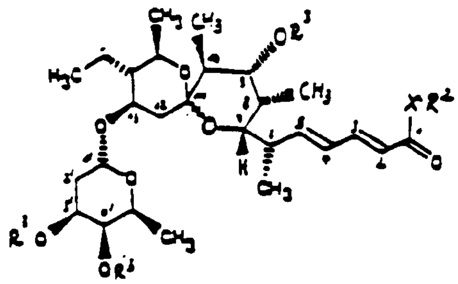

III

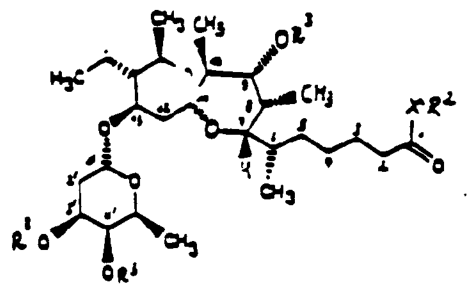

III'

in which the C=C double bonds in the 2,3 and 4,5 position may also be hydrogenated (formulae (I') and (III')) and in which

$R^1$ is hydrogen or a radical of the formula (VI)

$$-(CH_2)_n-R^4 \qquad (VI)$$

in which

n = 1 to 3 and

$R^4$ is hydrogen, $C_1$-$C_{15}$-alkyl, $C_2$-$C_{15}$-alkenyl, $C_2$-$C_{15}$-alkynyl, $C_3$-$C_9$-cycloalkyl, phenyl or a heteroaryl having 3 to 9 ring atoms, where the heterocycle is optionally substituted by chlorine, bromine, iodine, nitro, hydroxyl or $C_1$-$C_4$-alkyl or $C_1$-$C_4$-alkoxy,

X is 0 or, if $R^1$ is not hydrogen and the C=C double bonds in formula (I) and (III) are hydrogenated in the 2,3 and 4,5 position, is also NH,

$R^2$ is hydrogen or $C_1$-$C_4$-alkyl or, if X is NH, is a radical of the formula VI, where n and $R^4$ have the meanings indicated above for formula VI and in which

$R^3$ is hydrogen or a radical of the formula (II)

$$-\overset{\overset{\displaystyle O}{\|}}{C}-(CH_2)_n-R^4 \qquad (II)$$

in which n and $R^4$ have the meanings indicated above for formula VI.

2. An elaiophylin derivative of the formula (I) or (III) as claimed in claim 1, in which

$R^4$ is hydrogen, $C_1$-$C_6$-alkyl, phenyl or naphthyl, thiophene, furan, pyridine, pyrimidine or pyrazine.

3. An elaiophylin derivative of the formula (I) or (III) as claimed in claim 1 and/or 2 in which

$R^4$ is hydrogen, $C_1$-$C_4$-alkyl or phenyl.

4. A process for the preparation of elaiophylin derivatives of the formula I or III, which comprises

a) reacting elaiophylin of the formula IV

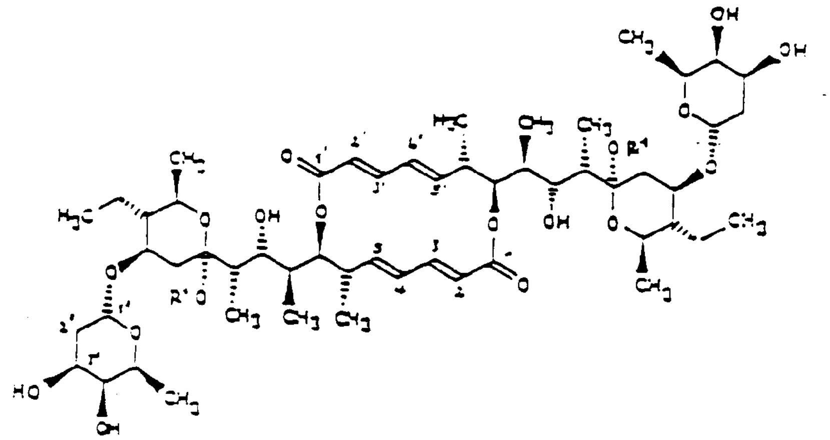

(IV)

in which $R^1$ is hydrogen, with an alcohol of the formula $HO-(CH_2)_n-R^4$, in which n and $R^4$ have the meaning indicated in claim 1, to give a compound of the formula IV in which $R^1$ is $(CH_2)_n-R^4$, where n and $R^4$ have the meanings indicated in claim 1 and first either reducing the intermediate thus obtained, if appropriate after its isolation,

b) in the presence of hydrogenation catalysts to give the compound of the formula IV′

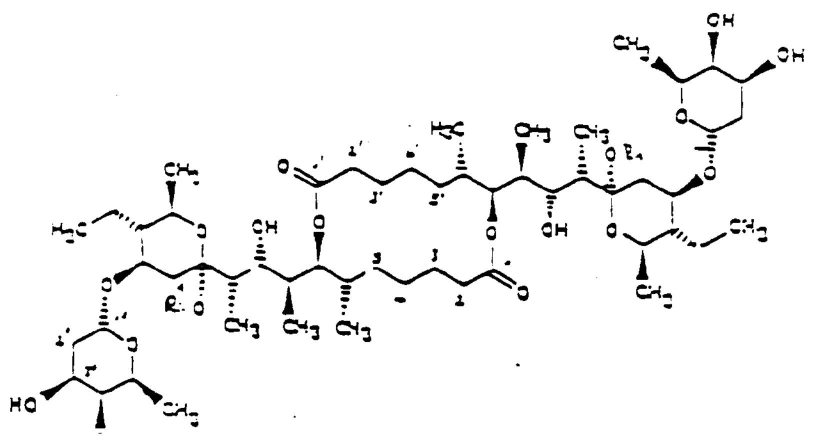

(IV')

in which the C=C double bonds in the 2,3 and 4,5 and also the 2′,3′ and 4′,5′ position are hydrogenated and $R^1$ has the meaning indicated in a), or

c) reacting the intermediate obtainable by reaction a) or the intermediate obtainable by reaction b) with alkali metal alcoholates of the formula $R^2O^-M^+$, in which M is lithium, sodium or potassium and $R^2$ has the meaning indicated in claim 1, to give compounds of the formula I or I″, in which $R^3$ is hydrogen, $XR^2$ is a radical $OR^2$ and $R^1$ and $R^2$ have the meanings indicated in claim 1 in addition to hydrogen, or

d) reacting a compound obtained by c) in a non-aqueous solvent with a LEWIS acid, to give a compound of the formula III, in which $XR^2$ and $R^3$ have the meaning indicated in c), or

e) reducing a compound obtained by c) or d) with hydrogen in the presence of hydrogenation catalysts to give compounds of the formula I′ or III′, in which $R^1$, $XR^2$ and $R^3$ have the meaning indicated in c) or d), or

f) reducing a compound obtained by c) with hydrogenation catalysts which additionally exhibit LEWIS acid activity to give a compound obtained by e), or

g) reacting a compound obtained by c) or d) with ammonia or a primary amine of the formula $H_2N-(CH_2)_n-R^4$, in which n and $R^4$ have the meanings indicated in claim 1, to give compounds of the formula I′ or III′, in which X is NH, or

h) reacting a compound obtained by c), d) or g) with a compound of the formula $Z-CO-(CH_2)-R^4$ in which n and $R^4$ have the meaning indicated in claim 1 and in which Z is a nucleofugic group, to give a compound of the formulae (I) or (III) or (I′) or (III′), in which $R^1$, $XR^2$ and $R^3$ have the meanings indicated in claim 1, or

i) reacting a compound of the formula (I) or (I′), in which $R^1$ and $R^2$ except for hydrogen, and also $R^3$ have the meaning indicated in claim 1, with a LEWIS acid in the presence of an aqueous solvent, a compound of the formula (I) or (I′) being obtained, in which $R^1$ is hydrogen and $XR^2$ and $R^3$ remain unchanged com-

pared to the starting compound (I) or (I′) for the reaction i).

5. A process as claimed in claim 4, wherein elaiophylin (formula IV, $R^1 = R^2 = R^3 = H$) is first hydrogenated according to reaction b) from claim 4 to give the compound (IV′), then reacted according to reaction a) with an alcohol of the formula $HO-(CH_2)_n-R^4$, in which n and $R^4$ have the meanings indicated in claim 1, to give a compound of the formula (IVa′).

6. An elaiophylin derivative as claimed in claim 1 for use as a pharmaceutical.

7. An elaiophylin derivative as claimed in claim 1 for use as an anthelmintic.

8. A pharmaceutical having anthelmintic action containing an anthelmintically acting amount of one or more different compounds of the formulae (I) and/or (III) as claimed in claim 1 and, if appropriate, pharmacologically tolerable auxiliaries or/and excipients and optionally other active compounds.

## Revendications

1. Dérivés de l'élaiophyline de formules (I) et (III)

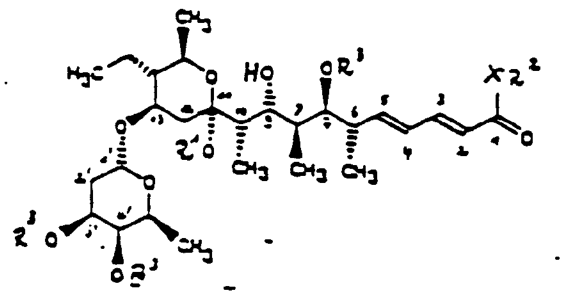

I

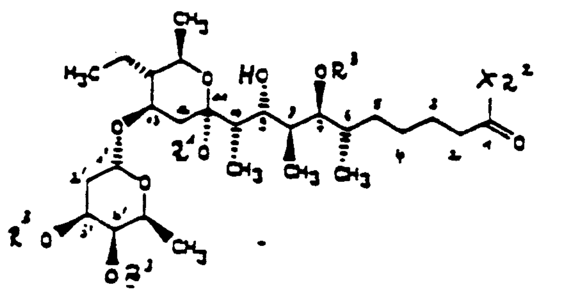

I′

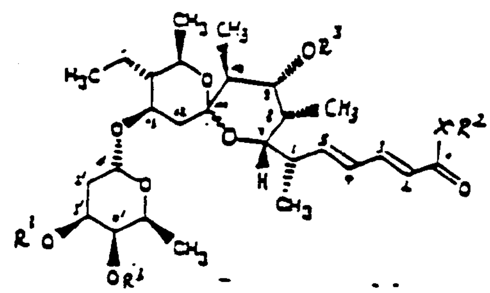

III

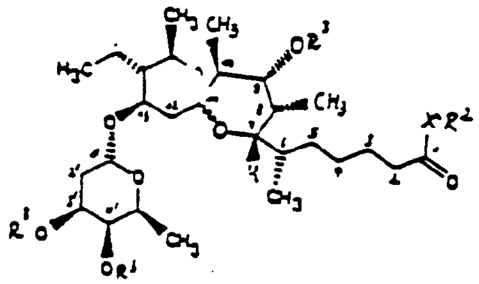

III'

dans lesquelles les doubles liaisons C=C en positions 2,3 et 4,5 peuvent également être hydrogénées [formules (I') et (III')] et dans lesquelles
$R^1$ représente un atome d'hydrogène ou un radical de formule (VI)

$-(CH_2)_n-R^4$ (VI)

dans laquelle
n = 1 à 3 et
$R^4$ est un atome d'hydrogène, un groupe alkyle en $C_1$-$C_{15}$, alcényle en $C_2$-$C_{15}$, alcynyle en $C_2$-$C_{15}$, cycloalkyle en $C_3$-$C_9$, phényle ou un radical hétéroaryle ayant de 3 à 9 atomes formant le cycle, les composés cycliques étant éventuellement substitués par des atomes de chlore, brome, iode ou des groupes nitro, hydroxy, alkyle en $C_1$-$C_4$ ou alcoxy en $C_1$-$C_4$,
X représente 0 ou, lorsque $R^1$ n'est pas un atome d'hydrogène et les doubles liaisons C=C dans les formules (I) et (III) sont hydrogénées en positions 2,3 et 4,5, également NH,
$R^2$ est un atome d'hydrogène ou un groupe alkyle en $C_1$-$C_4$, ou, si X est NH, un radical de formule (VI), n et $R^4$ ayant les significations données plus haut à propos de la formule (VI), et
$R^3$ représente un atome d'hydrogène ou un radical de formule (II)

$$-\overset{O}{\overset{\|}{C}}-(CH_2)_n-R^4 \qquad (II)$$

dans laquelle n et $R^4$ ont les significations données plus haut à propos de la formule (VI).

2. Dérivés d'élaiophyline de formules (I) et (III) selon la revendication 1, dans lesquels
$R^4$ est un atome d'hydrogène, un groupe alkyle en $C_1$-$C_6$, phényle ou naphtyle, thiophène, furanne, pyridino, pyrimidino ou pyrazino.

3. Dérivés d'élaiophyline de formules (I) et (III) selon la revendication 1 et/ou la revendication 2, dans lesquels
$R^4$ est un atome d'hydrogène ou un groupe alkyle en $C_1$-$C_4$ ou phényle.

4. Procédé pour la préparation de dérivés d'élaiophyline de formules (I) et (III), caractérisé en ce que
a) on fait réagir l'élaiophyline de formule (IV)

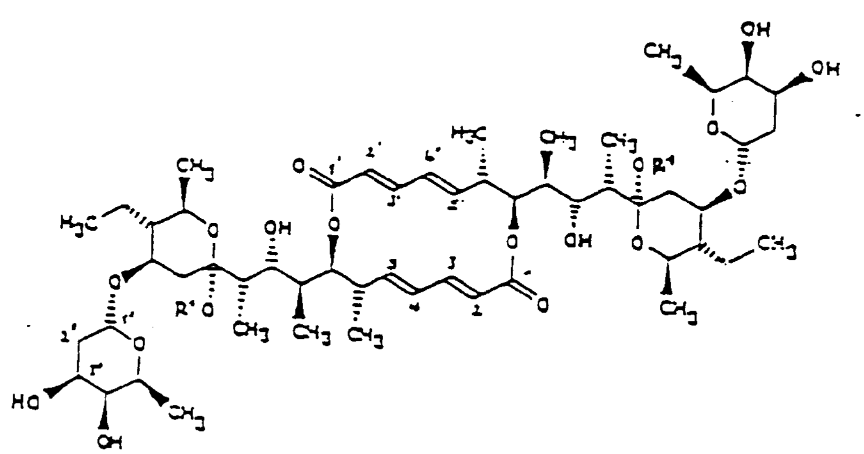

(IV)

dans laquelle $R^1$ représente un atome d'hydrogène, avec un alcool de formule $HO-(CH_2)_n-R^4$, dans laquelle n et $R^4$ ont les significations données dans la revendication 1, pour aboutir à un composé de formule (IV), dans laquelle $R^1$ représente $(CH_2)_n-R^4$, n et $R^4$ ayant les significations données dans la revendication 1, et soit

b) on réduit d'abord le produit intermédiaire ainsi obtenu, éventuellement après l'avoir isolé, en présence de catalyseur d'hydrogénation, pour aboutir au composé de formule (IV')

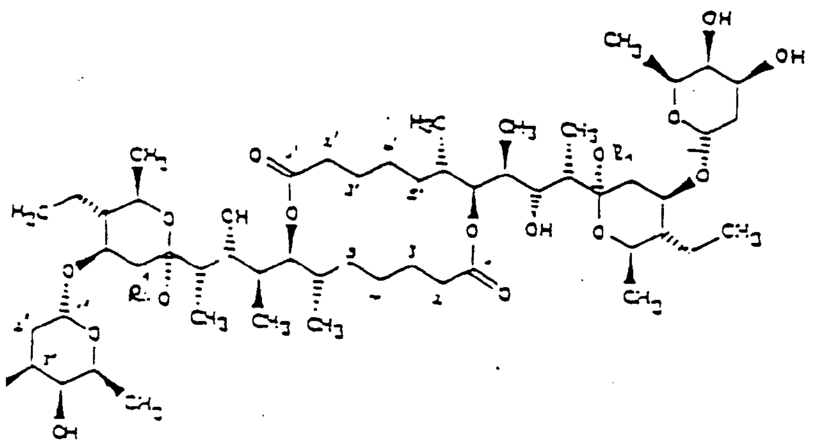

(IV')

dans laquelle les doubles liaisons C=C en positions 2,3 et 4,5 ainsi que 2',3' et 4',5' sont hydrogénées et $R^1$ a la signification donnée en a), soit

c) on fait réagir le produit intermédiaire pouvant être obtenu après la réaction a) ou le produit intermédiaire pouvant être obtenu après la réaction b), avec des alcoolates de métaux alcalins de formule $R^2O^-M^+$, dans laquelle M représente le lithium, le sodium ou le potassium et $R^2$ a la signification donnée dans la revendication 1, pour aboutir aux composés de formule (I) ou (I') dans lesquels $R^3$ est un atome d'hydrogène, $XR^2$ représente un radical $OR^2$, et $R^1$ et $R^2$, en plus de celle d'un atome d'hydrogène, ont les significations données dans la revendication 1, ou

d) on fait réagir avec un acide de Lewis un composé obtenu selon c), dans un solvant non aqueux, pour aboutir à un composé de formule (III) dans lequel $XR^2$ et $R^3$ ont les significations données en c), ou

e) on réduit un composé obtenu selon c) ou d), avec de l'hydrogène en présence de catalyseurs d'hydrogénation, pour aboutir à des composés de formule (I') ou (III'), dans lesquels $R^1$, $XR^2$ et $R^3$ ont les significations données en c) ou d), ou

f) on réduit un composé obtenu selon c) avec des catalyseurs d'hydrogénation qui ont en outre une activité d'acide de Lewis, pour aboutir à un composé obtenu selon e), ou g) on fait réagir un composé obtenu selon c) ou d) avec l'ammoniac ou une amine primaire de formule $H_2N-(CH_2)_n-R^4$, dans laquelle n et $R^4$ ont les significations données dans la revendication 1, pour aboutir à des composés de formule (I') ou (III') dans lesquels X représente NH, ou

h) on fait réagir un composé obtenu selon c), d) ou g), avec un composé de formule $Z-CO-(CH_2)-R^4$, dans laquelle n et $R^4$ ont les significations données dans la revendication 1 et dans laquelle Z représente un groupe nucléofuge, pour aboutir à un composé de formules (I) ou (III) ou, respectivement, (I') ou (III'), dans lesquelles $R^1$, $XR^2$ et $R^3$ ont les significations données dans la revendication 1, ou

i) on fait réagir avec un acide Lewis un composé de formule (I) ou (I'), formules dans lesquelles $R^1$ et $R^2$, jusqu'à celle d'un atome d'hydrogène, ainsi que $R^3$, ont les significations données dans la revendication 1, en présence d'un solvant aqueux, pour obtenir un composé de formule (I) ou (I') dans lequel $R^1$ représente un atome d'hydrogène, et $XR^2$ et $R^3$ restent inchangés par rapport aux composés de départ (I) ou (I') pour la réaction i).

5. Procédé selon la revendication 4, caractérisé en ce que l'on soumet d'abord à une hydrogénation l'élaiophyline [formule (IV), $R^1 = R^2 = R^3 = H$] selon la réaction b) de la revendication 4, pour aboutir au composé (IV'), puis on la fait réagir selon la réaction a) avec un alcool de formule $HO-(CH_2)_n-R^4$, dans laquelle n et $R^4$ ont les significations données dans la revendication 1, pour aboutir à un composé de formule (IVa').

6. Dérivés d'élaiophyline selon la revendication 1, pour utilisation en tant que médicaments.

7. Dérivés d'élaiophyline selon la revendication 1, pour utilisation en tant qu'anthelminthiques.

8. Médicaments à activité anthelminthique, contenant une quantité à action anthelminthique d'un ou plusieurs composés différents de formule(s) (I) et/ou (III) selon la revendication 1 et éventuellement des adjuvants

et/ou véhicules pharmacologiquement acceptables et éventuellement d'autres substances actives.